# EUROPEAN PATENT SPECIFICATION

(11) **EP 1 268 477 B1**
(45) Date of publication and mention of the grant of the patent: **21.04.2010**
(21) Application number: 01909799.7
(22) Date of filing: 28.02.2001
(51) Int. Cl.: C07D 471/04, A61K 31/44, A61P 29/00

(54) **SUBSTITUTED BETA-CARBOLINES WITH IKB-KINASE INHIBITING ACTIVITY**
SUBSTITUIERTE BETA-CARBOLINE MIT IKB-KINASE HEMMENDER WIRKUNG
BETA-CARBOLINES SUBSTITUEES AYANT UNE ACTIVITE INHIBITRICE DE L'IKB-KINASE

(30) Priority: 15.03.2000 EP 00105514; 18.11.2000 EP 00125169
(43) Date of publication of application: 02.01.2003
(73) Proprietor: Sanofi-Aventis Deutschland GmbH, 65929 Frankfurt am Main (DE)
(72) Inventor: RITZELER, Olaf, 65931 Frankfurt am Main (DE); CASTRO, Alfredo, Winchester, MA 01890 (US); GRENIER, Louis, Medford, MA 02155 (US); SOUCY, Francois, Medford, MA 02155 (US); HANCOCK, Wayne, W., Medfield, MA 02052 (US); MAZDIYASNI, Hormoz, Douglas, MA 01592 (US); PALOMBELLA, Vito, Needham, MA 02192 (US); ADAMS, Julian, Chestnut Hill, MA 02167 (US)
(86) International application number: PCT/EP2001/002237
(87) International publication number: WO 2001/068648

(56) References cited:
- EP-A- 1 048 666
- DE-A- 19 807 993
- DE-C- 568 675
- US-A- 4 631 149
- US-A- 5 854 003
- CHEMICAL ABSTRACTS, vol. 74, no. 1, 4 January 1971 (1971-01-04) Columbus, Ohio, US; abstract no. 546, HO ET AL.: "Inhibitors of monoamine oxidase..." XP002165158
- COULTHARD C E ET AL: "THE CHEMOTHERAPY OF DERIVATIVES OF HARMINE AND HARMALINE I" BIOCHEMICAL JOURNAL,PORTLAND PRESS, LONDON,GB, vol. 27, no. 3, 1933, pages 727-739, XP000917474 ISSN: 0264-6021
- KONOWALOWA R ET AL: "UNTERSUCHUNGEN IN DER REIHE DES HARMINS UND HARMALINS. 2. MITTEILUNG1): NITRO= UND AMINODERIVATES DES HARMINS UND HARMALINS, O= ALKYLAETHER DES HARMOLS UND HARMALOLS" ARCHIV DER PHARMAZIE,VCH VERLAGSGESELLSCHAFT MBH, WEINHEIM,DE, vol. 273, November 1934 (1934-11), pages 156-163, XP000917794 ISSN: 0365-6233
- DATABASE RTECS [Online] RTECS NUMBER UU9354600, July 1986 (1986-07) XP002170076
- CHEMICAL ABSTRACTS, vol. 96, no. 9, 1 March 1982 (1982-03-01) Columbus, Ohio, US; abstract no. 69273, SULTANA ET AL.: "Synthetic studies..." XP002170075

## Description

The invention relates to novel substituted beta-carbolines, a process for their preparation and use thereof as pharmaceuticals.

United States Patent 4,631,149 discloses beta-carbolines useful as antiviral, antibacterial and antitumor agents. United States Patent 5,604,236 discloses beta-carboline derivatives containing an acidic group, useful as thromboxane syntheses inhibitors.

NFkB is a heterodimeric transcription factor which can activate a large number of genes which code, inter alia, for proinflammatory cytokines such as IL-1, IL-2, TNFα or IL-6. NFkB is present in the cytosol of cells, building a complex with its naturally occurring inhibitor IkB. The stimulation of cells, for example by cytokines, leads to the phosphorylation and subsequent proteolytic degradation of IkB. This proteolytic degradation leads to the activation of NFkB, which subsequently migrates into the nucleus of the cell and there activates a large number of proinflammatory genes.

In disorders such as rheumatoid arthritis (in the case of inflammation), osteoarthritis, asthma, cardiac infarct, Alzheimer's disease or atherosclerosis, NFkB is activated beyond the normal extent. Inhibition of NFkB is also of benefit in cancer therapy, since it is employed there for the reinforcement of the cytostatic therapy. It was possible to show that pharmaceuticals such as glucocorticoids, salicylates or gold salts, which are employed in rheumatic therapy, intervene in an inhibitory manner at various points in the NFkB-activating signal chain or interfere directly with the transcription of the genes. The first step in the signal cascade mentioned is the degradation of IkB. This phosphorylation is regulated by the specific IkB kinase. To date, no inhibitors are known which specifically inhibit IkB kinase.

In the attempt to obtain active compounds for the treatment of rheumatoid arthritis in the case of inflammation, osteoarthritis, asthma, cardiac infarct, Alzheimer's disease, carcinomatous disorders for potentiation of cytotoxic therapies or atherosclerosis, it has now been found that the benzimidazoles according to the invention are strong and very specific inhibitors of IkB kinase.

The invention therefore relates to the use of the compounds of the formula 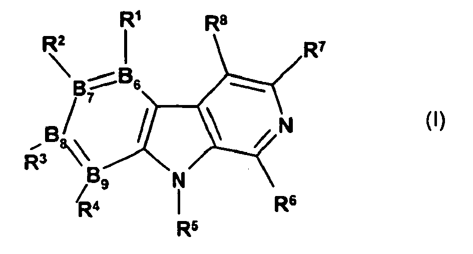 and/or a stereoisomeric form of the compounds of the formula I and/or a physiologically tolerable salt of the compounds of the formula I,
for the production of pharmaceuticals for the prophylaxis and therapy of disorders in whose course an increased activity of IₖB kinase is involved selected from asthma, osteoarthritis, rheumatoid arthritis in the case of inflammation, Alzheimer's disease, carcinomatous disorders for potentiation of cytotoxic therapies, cardiac infarct or allograft rejection,
wherein B₆ B₇, B₈ and B₉ are independently selected from the group consisting of carbon atom and nitrogen atom and wherein B₆, B₇, B₈ and B₉ together are no more than two nitrogen atoms at the same time; where the substituents R¹ R², R³ and R⁸ independently of one another are
1. hydrogen atom,
2. halogen,
3. -OH,
4. -CN,
5. sulfo,
6. -NO,
7. -NH₂,
8. alkoxy,
9. substituted amino,
10. -NH-C(O)-R¹⁵, wherein R¹⁵ is a heterocycle having 5 to 7 ring atoms, alkyl, aryl, substituted aryl or substituted alkyl,
11. -COOH,
12. -OR¹⁰, wherein R¹⁰ is alkyl, substituted alkyl or aryl,
13. -C(O)-R¹², wherein R¹² is alkyl, substituted alkyl or aryl,
14. -C(O)-O-R¹², wherein R¹² is as defined above,
15. aryl,
16. -O-aryl,
17. substituted aryl,
18. -O-substituted aryl,
19. alkyl,
20. substituted alkyl,
21. -CF₃ or
22. -CF₂-CF₃,
R4 is
1. -OH,
2. -CN,
3. sulfo,
4. -NH₂,
5. alkoxy,
6. substituted amino,
7. -NH-C(O)-R¹⁵, wherein R¹⁵ is a heterocycle having 5 to 7 ring atoms, alkyl, aryl, substituted aryl or substituted alkyl,
8. -COOH,
9. -O-R¹⁰, wherein R¹⁰ is alkyl, substituted alkyl or aryl,
10. -C(O)-O-R¹², wherein R¹² is as defined above,
11. aryl,
12. -O-aryl,
13. substituted aryl,
14. -O-substituted aryl,
15. alkyl,
16. substituted alkyl,
17. -CF₃ or
18. -CF₂-CF₃,
provided that at least one of R¹, R², R³, R⁴ and R⁸ is not a hydrogen atom,
R⁵ is
1. hydrogen atom,
2. alkyl,
3. alkyl radical, substituted at one or more positions by one or more of the radicals, halogen, amino or hydroxyl,
4. -C(O)-R⁹ or
5. -S(O)₂-R⁹, in which
R⁹ is
a) alkyl,
b) alkyl radical, substituted at one or more positions by one or more of the radicals, halogen, amino or hydroxyl,
c) aryl,
d) aryl radical, substituted at one or more positions by one or more of the radicals, halogen, amino, or hydroxyl,
e) -NH₂,
f) alkoxy or
g) substituted amino, and
R⁶ and R⁷ independently of one another are
1. hydrogen atom,
2. halogen,
3. -OH,
4. methyl,
5. -O-(C₁-C₁₀)-alkyl, wherein alkyl is unsubstituted or mono- to trisubstituted independently of one another by
   - 5.1: aryl,
   - 5.2: halogen,
   - 5.3: -NO₂,
   - 5.4: sulfo,
   - 5.5: -COOH,
   - 5.6: -NH₂,
   - 5.7: -O-(C₁-C₄)-alkyl or
   - 5.8: -OH, or
6. N(R¹³)₂ , wherein R¹³ is independently of one another hydrogen atom, aryl, -C(O)-(C₁-C₄)-alkyl or substituted aryl or alkyl, wherein alkyl is unsubstituted or mono- to tri- substituted independently of one another as defined under 5.1 to 5.8, or R¹³ together with the nitrogen atom to which it is bonded form a heterocycle having 5 to 7 ring atoms,
wherein halogen means fluorine, chlorine, bromine or iodine,
sulfo means -S(O)y-R¹⁴ , wherein R¹⁴ is an alkyl, aryl, substituted aryl, substituted alkyl, amino, or substituted amino and y is zero, one or two, alkyl means a straight or branched chain hydrocarbon radical having 1 to 10 carbon atoms,
aryl means an organic radical derived from an aromatic molecule by removal of one atom selected from phenyl, pyridyl, thiazolyl and naphthyl,
substituted aryl means an aryl radical substituted at one or more positions by one or more radicals of the group halogen, alkyl, substituted alkyl, nitro, sulfo, amino,
substituted alkyl means an alkyl radical substituted at one or more positions by one or more radicals of the group halogen, nitro, sulfo, amino, substituted amino, carboxyl, alkoxy, -O-aryl, -O-substituted aryl, and hydroxyl,
substituted amino means a -N(R¹³)₂ wherein R¹³ is independently of one another hydrogen atom, sulfo, alkyl, aryl, -C(O)-alkyl, C(O)-NH-aryl, -C(O)-O-aryl, -C(O)-O-alkyl, or C(O)-O-aryl, wherein each alkyl or aryl may be independently substituted,
alkoxy means -O-alkyl or -O-substituted alkyl, and
heterocycle having 5 to 7 ring atoms means a radical of a monocyclic saturated system having 5 to 7 ring members, which contains 1, 2 or 3 heteroatoms selected from N, O and S as ring members.

The invention further relates to the use of a compound of formula II 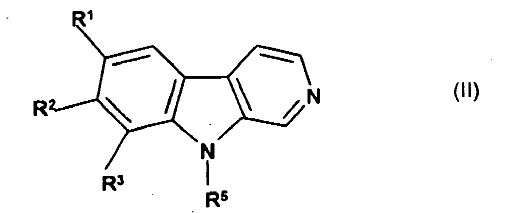 and/or a stereoisomeric form of the compounds of the formula II and/or a physiologically tolerable salt of the compounds of the formula II,
for the production of pharmaceuticals for the prophylaxis and therapy of disorders in whose course an increased activity of IₖB kinase is involved selected from asthma, osteoarthritis, rheumatoid arthritis in the case of inflammation, Alzheimer's disease, carcinomatous disorders for potentiation of cytotoxic therapies, cardiac infarct or allograft rejection, wherein;
R¹ and R² are independently from one another hydrogen atom, halogen, cyano, amino, -O-(C₁-C₄)-alkyl, nitro, -CF₃ or N(R¹³)₂,
   wherein R¹³ is independently of one another hydrogen atom, -(C₁-C₇)-alkyl, -C(O)-(C₁-C₇)-alkyl, -C(O)-pyridyl, -C(O)-phenyl or -C(O)-O-(C₁-C₄)-alkyl, wherein alkyl or phenyl are unsubstituted or mono- to tri- substituted independently of one another by halogen or -O-(C₁-C₄)-alkyl,
R³ is cyano, amino, -O-(C₁-C₄)-alkyl, -CF₃ or N(R¹³)₂,
   wherein R¹³ is independently of one another hydrogen atom, -(C₁-C₇)-alkyl, -C(O)-(C₁-C₇)-alkyl, -C(O)-pyridyl, -C(O)-phenyl or -C(O)-O-(C₁-C₄)-alkyl, wherein alkyl or phenyl are unsubstituted or mono- to tri- substituted independently of one another by halogen or -O-(C₁-C₄)-alkyl, and
R⁵ is hydrogen atom, -C(O)-CH₃, methyl, -S(O)₂-CH₃, -C(O)-morpholinyl, -CH₂-CH₂-OH or -CH₂-C(O)-NH₂,
provided that no more than two of R¹, R², R³ and R⁵ are a hydrogen atom.

The invention further relates to a process for the preparation of the compounds of the formula I and/or a stereoisomeric form of the compounds of the formula I and/or of a physiologically tolerable salt of the compounds of the formula I, which comprises
a) reacting a compound of formula III 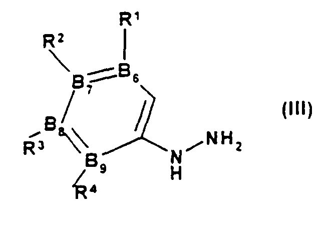 in which R¹, R², R³, R⁴, B₆, B₇, B₈ and B₉ are each as defined in formula I, with a compound of the formula IV, 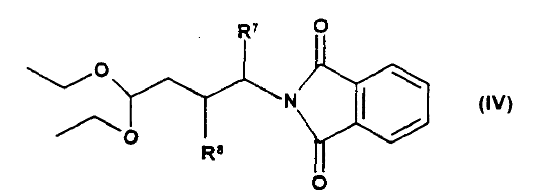 in the presence of a acid, converting into a compound of the formula V, 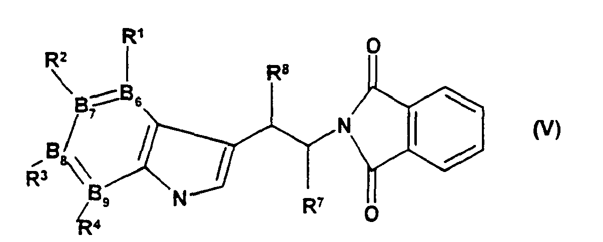 and is reacted with hydrazine hydrate and later with formaldehyde (R⁶ is H) or R⁶CHO to give a compound of formula VI 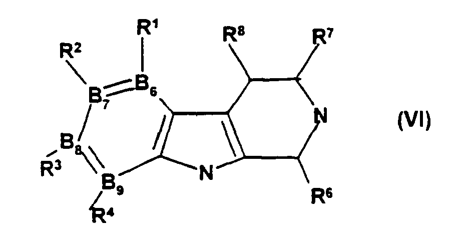 and oxidized to give a compound of the formula VII, 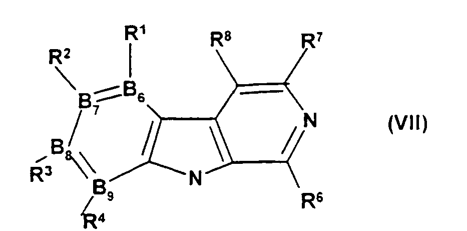 where R¹ to R⁴ and B₆ to B₉ are as defined in formula I and R⁵ is hydrogen atom, or
b) a compound of the formula VII is reacted with a compound of the formula VIII

   Y-R⁵ (VIII)

   where Y is halogen or -OH and R⁵ is as defined in formula I, to give a compound of the formula I, or
c) resolving a compound of the formula I, which on account of its chemical structure occurs in enantiomeric forms, prepared by process a) or b) into the pure enantiomers by salt formation with enantiomerically pure acids or bases, chromatography on chiral stationary phases or derivatization by means of chiral enantiomerically pure compounds such as amino acids, separation of the diastereomers thus obtained, and removal of the chiral auxiliary groups, or
d) isolating the compound of the formula I prepared by process a), b) or c) either in free form or, in the case of the presence of acidic or basic groups, converting it into physiologically tolerable salts.

The preparation of physiologically tolerable salts of compounds of the formula I capable of salt formation, including their stereoisomeric forms, is carried out in a manner known per se. With basic reagents such as hydroxides, carbonates, hydrogencarbonates, alkoxides and also ammonia or organic bases, for example trimethyl- or triethylamine, ethanolamine or triethanolamine or alternatively basic amino acids, for example lysine, ornithine or arginine, the carboxylic acids form stable alkali metal, alkaline earth metal or optionally substituted ammonium salts. If the compounds of the formula I contain basic groups, stable acid addition salts can also be prepared using strong acids. For this, both inorganic and organic acids such as hydrochloric, hydrobromic, sulfuric, phosphoric, methanesulfonic, benzenesulfonic, p-toluenesulfonic, 4-bromobenzenesulfonic, cyclohexylamidosulfonic, trifluoromethylsulfonic, acetic, oxalic, tartaric, succinic or trifluoroacetic acid are suitable.

The pharmaceuticals according to the invention are in general administered orally or parentally or by rectal, inhale or transdermal administration.

On account of the pharmacological properties, the compounds according to the invention are suitable for the prophylaxis and therapy of all those disorders in whose course an increased activity of IkB kinase is involved. Disorders in whose course an increased activity of IkB kinase is involved include, for example asthma, osteoarthritis, rheumatoid arthritis in the case of inflammation, Alzheimer's disease, carcinomatous disorders for potentiation of cytotoxic therapies, cardiac infarct or allograft rejection.

Suitable solid or pharmaceutical preparation forms are, for example, granules, powders, coated tablets, tablets, (micro)capsules, suppositories, syrups, juices, suspensions, emulsions, drops or injectable solutions, and preparations having protracted release of active compound, in whose preparation customary auxiliaries, such as excipients, disintegrants, binders, coating agents, swelling agents, glidants or lubricants, flavourings, sweeteners and solubilizers are used. Frequently used auxiliaries which may be mentioned are magnesium carbonate, titanium dioxide, lactose, mannitol and other sugars, talc, lactoprotein, gelatin, starch, cellulose and its derivatives, animal and vegetable oils such as cod liver oil, sunflower, groundnut or sesame oil, polyethylene glycol and solvents such as, for example, sterile water and mono- or polyhydric alcohols such as glycerol.

The pharmaceutical preparations are preferably produced and administered in dose units, each unit containing as active constituent a certain dose of the compound of the formula I according to the invention. In the case of solid dose units such as tablets, capsules, coated tablets or suppositories, this dose can be up to approximately 1000 mg, preferably from approximately 50 mg to 300 mg and in the case of injection solutions in ampoule form up to approximately 300 mg, preferably from approximately 10 mg to 100 mg.

For the treatment of an adult patient weighing approximately 70 kg, depending on the efficacy of the compound according to formula I, daily doses of approximately 20 mg to 1000 mg of active compound, preferably from approximately 100 mg to 500 mg, are indicated. Under certain circumstances, however, even higher or lower daily doses may be appropriate. The administration of the daily dose can be carried out both by single administration in the form of an individual dose unit or else of a number of smaller dose units and by multiple administration of subdivided doses at specific intervals.

As a rule, final products are determined by mass-spectroscopic methods (FAB-, ESI-MS). Temperatures are given in degrees Celsius, RT means room temperature (22 °C to 26 °C). Abbreviations used are either explained or correspond to the customary conventions.

### Example 1, 7-bromo-β-carboline

A solution of norharmane (600 mg, 3.57 mmol) in tetrahydrofuran (THF; 50 ml) was treated with bromine (0.40 ml, 7.80 mmol) at RT while stirring. After stirring for 18 h at RT, the reaction was concentrated under reduced pressure and the resulting residue was sonicated in 10% aqueous Na₂CO₃ (100 ml). The product was filtered and washed with water to give 905 mg of crude product. The crude product was crystallized from xylenes to provide in two crops 580 mg of 7-bromo-β-carboline.

### Example 2, 1-acetyl-7-bromo-β-carboline

A solution of Example 1 (25 mg, 0.1 mmol) in dimetylformamide (DMF; 2 ml) was treated with 0.10 ml of 1 M aqueous NaOH (0.10 mmol). After stirring at RT for 30 minutes, acetic anhydride (0.010 ml, 0.095 mmol) was added and reaction stirred 18 h at RT. The reaction was partitioned in EtOAc and 5% citric acid and the organic layer washed with water, dried (brine; MgSO4), and concentrated to give 23 mg of crude product. The crude product was chromatographed (7:3 hexane-acetone) on silica gel to give 10 mg of 1-acetyl-7-bromo-β-carboline.

### Example 3, 7-fluoro-β-carboline

A mixture of 5-fluorotryptamine hydrochloride (200 mg, 0.93 mmol) in EtOAc (4 ml) and water (2 ml) was treated with glyoxalic acid hydrate (90 mg, 0.98 mmoi). The pH of the aqueous layer was adjusted to 5 (with 5% NaHCO₃ then 1 M HCl) and the mixture stirred vigorously at RT for 18 h. The mixture was then diluted with hexane (4 ml) and the product filtered and washed with water and (1:1) hexane-ethyl acetate.

The crude product from above in 6N HCl (3 ml) was treated 3 times with concentrated HCl (0.050 ml) every 15 min while refluxing. After refluxing for a total of 45 min, the reaction was concentrated to give a residue. The above residue was slurred in xylenes with triethylamine (0.40 ml, 2.9 mmol) and 10% Pd/C (200 mg). The mixture was refluxed for 1 h and then filtered hot through celite. The filtrate was concentrated and the residue chromatographed (5:95 methanol-chloroform) on silica gel to give 25 mg of 7-fluoro-β-carboline.

### Example 4, 7-isopropyl-β -carboline hydrochloride

A mixture of 4-isopropylphenylhydrazine hydrochloride (660 mg, 3.55 mmol) and 4-phthalimidobutanal diethyl acetal (1.15 g, 3.95 mmol) in Ethanol (EtOH; 30 ml) was heated at 60 °C to 65 °C for 1 h with water (0.050 ml). The mixture was then treated with concentrated HCl (0.50 ml) and refluxed for 14 h. After concentrating the reaction, the residue was partioned in methylene chloride and saturated aqueous NaHCO₃ The aqueous solution was extracted with methylene chloride (3 times) and the combined organic solutions were dried (MgSO₄) and concentrated to give after chromatography (4:1 hexane-ethyl acetate) on silica gel 146 mg of product.

The above product was treated with hydrazine hydrate (1 ml) in EtOH (4 ml) and water (1 ml) and stirred at RT overnight. After concentrating the reaction to an aqueous mixture, the product was extracted with methylene chloride (3 times). The combined organic solution was dried (MgSO₄) and concentrated. The residue was redissolved in methylene chloride and treated with 1 M HCl in ether. The precipitate was collected and washed with ether and hexane and dried to provide 102 mg of 6-isopropyltryptamine hydrochloride salt. This tryptamine obtained above was transformed into 7-Isopropyl- β -carboline according to the procedure used in example 3. The hydrochloride salt was obtained by treating a solution of 7-Isopropyl-β-carboline in methylene chloride with 1 M HCl in ether and concentrating. The residue was triturated with (1:1) methylene chloride-hexane to give 15 mg of 7-isopropyl-β-carboline hydrochloride.

### Example 5, 7-cyano- β -carboline

A dark solution of example 1 (190 mg, 0.62 mmol) and CuCN (110 mg, 1.22 mmol) in N-methyl 2-pyrrolidone (1.5 ml) was heated in a sealed reaction tube at 200 °C for 48 h. The mixture was filtered and the filtrate was diluted with water (50 ml). A brown solid that precipitated was filtered, washed with water, saturated aqueous NaHCO₃ and then methanol. This material was dissolved in DMSO and diluted with aqueous HCl. This homogeneous dark solution was decolorized with charcoal, filtered and concentrated to give a concentrated solution in DMSO. This DMSO solution was partitioned in (1:1) EtOAc-THF and saturated aqueous NaHCO₃. The organic solution was dried (MgSO₄) and concentrate to give 8 mg of 7-cyano- β -carboline.

### Example 6, 7-nitro-β- carboline hydrochloride

Norharmane (100 mg, 0.60 mmol) was treated with concentrated nitric acid (1.0 ml) and the resulting suspension was heated to 65 °C until the mixture became homogeneous (3 to 4 min). The solution was carefully poured into water (20 ml), and the precipitate filtered and washed with water then methanol. The solid was suspended in saturated aqueous NaHCO₃ and stirred vigorously before filtering and washing with water. The solid was taken up in hot methanol and this solution treated with 1 M HCl in ether (5 ml). The solution was concentrated and the residue triturated with ether to provide 58 mg of a 7:3 mixture of 7-nitro- β -carboline hydrochloride and 9-nitro- β-carboline hydrochloride.

### Example 7, 7-carboxy- β- carboline trifluoroacetat

Crude product from example 5 (from 210 mg of example 1, 0.85 mmol) was treated with 6 M HCl in a sealed reaction tube for 15 h at 110 °C. The reaction was evaporated to give a concentrated solution of product in N-methyl 2-pyrrolidone. A portion (half) was purified by preparative HPLC using a C₁₈ - packed column and eluting with a gradient (5:95 to 50:50) of water-acetonitril (with 0.1 % trifluoracetic acid). The pure fractions were combined and lyophilized to provide 11 mg of 7-carboxy- β -carboline trifluoroacetate.

### Example 8, 7,9-dibromo- β - carboline hydrochloride

A solution of the product from example 1 (140 mg, 0.58 mmol) in THF (2 ml) was treated with bromine (0.50 ml). After 10 min at RT, the reaction was diluted with chloroform and the product was filtered. The filtered product was taken up in methanol and treated with 1 M HCl in ether and concentrated. The residue was triturated with ether to provide 160 mg of 7,9-dibromo-β-carboline hydrochloride.

### Example 9, 7,9-dichloro-β-carboline

To a suspension of norharmane (84 mg, 0.50 mmol) in water (3 ml) at RT was added 1 M aqueous HCl (1.1 ml, 1.1 mmol). To this homogenous solution was added N-chlorosuccinimide (747 mg, 5.58 mmol) portionwise and the resulting solution was stirred at 60 °C to 70 °C for 3h. The reaction was partitioned in EtOAc and saturated aqueous NaHCO₃ and the organic layer was dried (brine; MgSO₄) and then concentrated. The residue was chromatographed (2:3 THF-hexane) on silica gel to give 24 mg of 7,9-dichloro-β-carboline after triturating with (1:1) methylene chloride-hexane, then with hexane.

### Example 10, 1-acetyl-7-bromo-β-carboline

To a suspension of NaH (95%, 14 mg, 0.60 mmol) in DMF (1.0 ml) at 5 °C to 10 °C was added the product from example 1 (74 mg, 0.30 mmol). The resulting mixture was stirred for 15 min at 5 °C to 10 °C before adding methanesulfonylchloride (0.030 ml, 0.38 mmol). The reaction was allowed to warm to RT and stirred for 2 h before partitioning into saturated aqueous NaHCO₃ and EtOAc. After stirring the mixture overnight, the organic layer was washed with water, dried (brine; MgSO₄), and concentrated. The residue was purified by chromatography (1:1 hexane-ethyl acetate) on silica gel to give 23 mg of 1-acetyl-7-bromo-β-carboline.

### Example 11, 7-bromo-1-methyl-β-carboline

To a suspension of NaH (95%, 6 mg, 0.24 mmol) in DMF (2.0 ml) at 5 °C to 10 °C was added the product from example 1 (50 mg, 0.20 mmol). The resulting mixture was stirred for 15 min at 5 °C to 10 °C before adding methyl-iodide (0.030 ml, 0.20 mmol) at 0 °C to 5 °C. The reaction stirred for 12 h at 0 °C to 5 °C before partitioning into water and EtOAc. The organic layer was washed with water, dried (brine; MgSO₄), and concentrated. The residue was purified by chromatography (gradient, 1:3 hexane-ethyl acetate to ethyl acetate) on silica gel to give 10 mg of 7-bromo-1-metyhl-β -carboline.

### Example 12, 7-chloro-β-carboline

To a solution of norharmane (2.0 g, 11.9 mmol) in water (89 ml) and 1 M aqueous HCl (29.8 ml, 29.8 mmol) was added N-chlorosuccinimide (3.17 g, 23.8 mmol) portionwise. The resulting solution was stirred at RT for 6 h, and then at 0 °C to 5 °C for 12 h. The reaction was diluted with water (100 ml) and basified cautiously with solid K₂CO₃ (4.3 g). After stirring at RT for 1 h, the product was collected and washed with water. The crude product was refluxed in chloroform for 1 h and filtered after cooling to 15 °C to provide 2.05 g of 7-chloro-β-carboline.

### Example 13, 1-acetyl-7-chloro-β-carboline

To a solution of the product from example 12 (104 mg, 0.50 mmol) in DMF (2.0 ml) at 3 °C to 5 °C was added NaH (95%, 15 mg, 0.625 mmol). The resulting mixture was stirred for 15 min before adding acetic anhydride (0.083 ml, 0.875 mmol). The reaction was allowed to warm to RT and stirred for 3 h before pouring into with water (25 ml). The slurry was stirred for 12 h, and the product collected to give after chromatography (1:3 hexane-ethyl acetate) on silica gel 82 mg of 1-acetyl-7-chloro-β-carboline.

### Example 14, 7-chloro-9-nitro-β-carboline

A mixture of the product from example 12 (500 mg, 2.48 mmol) in concentrated nitric acid (20 ml) was stirred at RT for 22 h. The reaction mixture was carefully poured into cold (3 °C to 5 °C) water (50 ml), and after stirring for 2 h the precipitate was collected. The solid was suspended in saturated aqueous NaHCO₃ (50 ml) and stirred at RT for 12 h. The product was filtered and washed with water to provide 550 mg of 7-chloro-9-nitro-β-carboline

### Example 15, 9-amino-7-chloro-β-carboline

To a suspension of the product from example 14 (548 mg, 2.22 mmol) in EtOH (14 ml) at 65 °C to 70 °C was added tin chloride dihydrate (2.5 g, 11.1 mmol). Thereafter, 6M aqueous HCl (14 ml) was added dropwise. The mixture was stirred at 70 °C to 80 °C for 3.5 h and then partitioned slowly into saturated aqueous NaHCO₃ (150 ml) and EtOAc (100 ml). The aqueous phase was extracted (2 times) and the combined organic solutions dried (brine; NaSO₄) and concentrated to give 484 mg of 9-amino-7-chloro-β-carboline.

### Example 16, 9-amino-7-chloro- β- carboline trifuoroacetate

To a solution of the product from example 15 (35 mg, 0.16 mmol) in pyridine (0.80 ml) was added acetic anhydride (0.018 ml, 0.19 mmol). The resulting mixture was allowed to stand at RT for 12 h before pouring into water (15 ml). The crude product was filtered and purified by preparative HPLC using a C₁₈ - packed column and eluting with a gradient (5:95 to 60:40) of water-acetonitril (with 0.1% trifluoracetic acid). The pure fractions were combined and lyophilized to provide 18 mg of 9-amino-7-chloro-β-carboline trifluoroacetate.

### Example 17, 7-bromo-1-carbonyl-(4'-morpholine)-β-carboline

A solution of the product from example 1 (125 mg, 0.51 mmol) in DMF (2 ml) was treated with 0.55 ml of 1 M aqueous NaOH (0.55 mmol). After stirring at RT for 30 minutes, 4-morpholinecarbonyl chloride (0.060 ml, 0.51 mmol) was added and reaction stirred 18 h at RT. The reaction was partitioned in EtOAc and 5% citric acid and the organic layer washed with water, dried (brine; MgSO₄), and concentrated. The residue was chromatographed (7:3 hexane-acetone) on silica gel to give 105 mg of 7-bromo-1-carbonyl-(4'-morpholino)-β -carboline.

### Example 18, 1-(2'-ethylacetate)-7-chloro-β-carboline

To a suspension of NaH (95%, 28 mg, 1.15 mmol) in DMF (1.0 ml) at 5 °C to 10°C was added the product from example 12 (202 mg, 1.0 mmol) in DMF (3 ml). The resulting mixture was stirred for 30 min at 5 °C to 10°C before adding ethyl bromoacetate (0.116 ml, 1.05 mmol). The reaction was allowed to be stirred for 1.5 h and then the reaction was diluted with saturated aqueous NaHCO₃ (30 ml). The product was extracted with EtOAc (30 ml; 2 times each 15ml), and the combined organic extracts were dried (brine; MgSO₄) then concentrated. The residue was purified by chromatography (1:3 hexane-ethyl acetate) on silica gel to give 270 mg of 1-(2'ethylacetate)-7-chloro-β-carboline.

### Example 19, 1-(2'-ethanoyl)-7-chloro-β-carboline

To a solution of the product from example 18 (50 mg, 0.17 mmol) in THF (1.7 ml) at 5 °C to 10 °C was added 1 M LAH in THF (0.17 ml, 0.17 mmol). The resulting mixture was stirred for 2 h at 5 °C to 10 °C before adding EtOAc (0.10 ml). The mixture was subsequently diluted with EtOAc (5 ml) and slowly treated with saturated aqueous NaHCO3 (5 ml). After diluting with water (10 ml) and brine (10 ml) the mixture was extracted with EtOAc. The organic solution was dried (brine; MgSO₄) then concentrated to give 42 mg of 1-(2'ethanol)-7-chloro-β-carboline.

### Example 20, 1-(2'-acetyl)-7-chloro-β-carboline

To a solution of the product from example 18 (107 mg, 0.37 mmol) in MeOH (3.7 ml) at RT was added water (3.7 ml) followed by treatment with 1 M aqueous NaOH (0.41 ml, 0.41 mmol). The resulting mixture was stirred for 2 h and the volatile removed under reduced pressure. The mixture was subsequently diluted with water (5 ml) and the pH adjusted to 5 to 6. The precipitate was filtered and washed with water to give 96 mg of 1-(2'-acetyl)-7-chloro-β-carboline.

### Example 21, 8-methoxy-β-carboline

Prepared from 6-methoxytryptamine using the procedure as in example 3.

### Example 22, see table 1 for structure

To a solution of the product from example 20 (59 mg, 0.23 mmol) in DMF (2.8 ml) at RT was added p-nitrophenol (40 mg, 0.29 mmol) followed by 1-ethyl-3-(3-dimethylaminopropyl)carbodiimide (48 mg, 0.25 mmol). The resulting mixture was stirred for 1.5 h at RT and then ammonium bicarbonate (55 mg, 0.69 mmol) was added. The reaction was stirred for 18 h at RT and then poured into water (20 ml). The aqueous mixture was basified to pH of about 10 with K₂CO₃. The precipitate was filtered and washed with water to give 47 mg of the title compound.

### Example 23, 8-hydroxy-2-methyl-β-carboline

A solution of harmine (616 mg, 2.9 mmol) in dichloroethane (20 ml) was treated with 2.0 ml of 1 M BBr₃ (4 mmol) in dichloroethane. The reaction was stirred at 60 °C for 48 h and then cooled to 0 °C before quenching with MeOH (5 ml). The reaction was concentrated and the residue triturated with methanol to give 413 mg of 8-hydroxy-2-methyl- β -carboline .

### Example 24, 6,8-dibromo-7-methoxy-β-carboline

A solution of the product from example 30 (90 mg, 0.45 mmol) in acetic acid (8 ml) was treated with bromine (0.025 ml, 0.48 mmol). The reaction was stirred at RT for 18 h and then concentrated. The residue was partitioned in EtOAc and aqueous NaHCO₃. The organic layer was dried (brine; MgSO₄) and concentrated. The residue was purified by chromatography (5:4 hexane-acetone) on silica gel to give 3 mg of 6,8-dibromo-7-methoxy-β-carboline.

### Example 25, see table 1 for structure

A solution of the product from example 23 (60 mg, 0.30 mmol) in DMF (3 ml) was treated with K₂CO₃ (100 mg) and t-butyl bromoacetate (0,040 ml, 0.27 mmol). After stirring at RT for 18 h, the reaction was partitioned in EtOAc and water. The organic layer was dried (brine; MgSO₄) and then concentrated. The residue was chromatographed (1:1 hexane-EtOAc) on silica gel to give 20 mg of the title compound.

### Example 26, see table for structure

A solution of the product from example 23 (50 mg, 0.25 mmol) in DMF (3 ml) was treated with K₂CO₃ (100 mg) and benzyl bromide (0,030 ml, 0.25 mmol). After stirring at RT for 18 h, the reaction was partitioned in EtOAc and water. The organic layer was dried (brine; MgSO₄) and then concentrated. The residue was chromatographed (1:1 hexane-EtOAc) on silica gel to give 12 mg of the title compound.

### Example 27, 7-ethoxy-2-methyl-β-carboline

A solution of the product from example 23 (60 mg, 0.30 mmol) in DMF (3 ml) was treated with K₂CO₃ (100 mg) and ethyl iodide (0,029 ml, 0.36 mmol). After stirring at RT for 18 h, the reaction was partitioned in EtOAc and water. The organic layer was dried (brine; MgSO₄) and then concentrated. The residue was chromatographed (1:1 hexane-acetone) on silica gel to give 20 mg of 7-ethoxy-2-methyl-β-carboline.

### Example 28, 7-bromo-2-methyl-β-carboline

Prepared from harmane using the same procedure as in example 1.

### Example 29, see table 1 for structure

A solution of the product from example 23 (60 mg, 0.30 mmol) in DMF (3 ml) was treated with K₂CO₃ (100 mg) and acetic anhydride (0,034 ml, 0.36 mmol). After stirring at RT for 18 h, the reaction was partitioned in EtOAc and water. The organic layer was dried (brine; MgSO₄) and then concentrated. The residue was chromatographed (1:1 hexane-acetone) on silica gel to give 18 mg of the title compound.

### Example 30, 7-methoxy-β-carboline

Prepared from 5-methoxytryptamine using the procedure in example 3.

### Example 31, 8-fluoro- β- carboline

Prepared from 6-fluorotryptamine using the same procedure as in example 3.

### Example 32, 7-bromo-2-methyl-8-methoxy- β - carboline

Prepared from harmine using the same procedure as in example 1.

### Example 33, 7-hydroxy-β-carboline

Prepared from the product of example 30 using the procedure in example 23.

### Example 34, 7-chloro-8-fluoro-β-carboline

Prepared from the product of example 31 using the procedure in example 12.

### Example 35, 7-methoxy-1-methyl-β-carboline

Prepared from the product of example 30 using the procedure in example 11.

### Example 36 9-chloro-8-methoxy- β -carboline trifluoroacetate and Example 37

7,9-dichloro-8- methoxy-β-carboline trifluoroacetate A solution of the product from example 21 (195 mg, 1.0 mmol) in 1 M HCl (3 ml) was treated with N-chlorosuccinimide (270 mg, 2 mmol) portionwise and the resulting solution was stirred at 60 °C to 70 °C for 3h. The reaction was evaporated and the crude product purified by preparative HPLC using a C₁₈-packed column and eluting with a gradient (5:95 to 50:50) of water-acetonitril (with 0.1 % trifluoroacetic acid). The pure fractions of each product were combined and lyophilized to provide 78 mg of 9-chloro-8-methoxy-β-carboline trifluoroacetate and 51 mg of 7,9-dichloro-8- methoxy-β-carboline trifluoroacetate.

### Example 38 7,9-dichloro-8-hydroxy-β-carboline

A mixture of the product of example 37 (590 mg, 2.21 mmol) in methylene chloride (25 ml) at 35 °C was treated with a solution of BBr₃ in methylene chloride (1 M, 6 ml, 6 mmol). After refluxing for 3h, the reaction was quenched with methanol (5 ml) and then concentrated. The residue was slurred in 60% NaHCO₃ solution, the product filtered and washed with water to give 500 mg of 7,9-dichloro-8-hydroxy-β-carboline.

### Example 39 7,9-dichloro-8-ethoxy-β-carboline

A mixture of the product of example 38 (35 mg, 0.14 mmol), K₂CO₃ (100 mg), and ethyl iodide (0.014 ml, 0.17 mmol) in acetone (5 ml) was stirred in a closed reaction tube at RT for 3 days. After concentrating the reaction, the residue was partitioned in ethyl acetate and water. The organic layer was dried (MgSO4) and concentrated to give crude product. The crude product was chromatographed (5% methanol in chloroform) on silica gel to give 8 mg of 7,9-dichloro-8-ethoxy-β-carboline.

### Example 40 7-chloro-8-fluoro-β-carboline trifluoroacetate

A solution of the product of example 31 (78 mg, 0.42 mmol) in 1 M HCl (1 ml) was treated with N-chlorosuccinimide (115 mg, 0.9 mmol) portionwise and the resulting mixture was stirred at 60 °C to 70 °C for 3h. The reaction was evaporated and the crude product purified by preparative HPLC using a C₁₈-packed column and eluting with a gradient (5:95 to 50:50) of water-acetonitril (with 0.1 % trifluoroacetic acid). The pure fractions with product were combined and lyophilized to provide 33 mg of the title compound.

### Example 41 1-hydroxy-7-trifluoromethyl-β-carboline and Example 42 7-trifluoromethyl-β-carboline

A solution of 3-hydroxy-2-piperdone (96 mg, 0.83 mmol) in methylene chloride (5 ml) was treated with Dess Martin reagent (352 mg, 0.85 mmol) at RT and the resulting mixture was stirred for 1 h. After filtering the salts from the reaction, the ketone in solution was treated with 4-trifluoromethyl-phenylhydrazine (145 mg, 0.83 mmol). After 15 min, hexane (20 ml) was added and the hydrazone collected by filtration. This crude hydrazone was heated at 95 °C in formic acid (70%, 10 ml) for 1 h. The reaction was evaporated and the residue was chromatographed (ethyl acetate) on silica gel to give 60 mg of dihydro 1-hydroxy-7-trifluoromethyl-β-carboline.

A portion of dihydro 1-hydroxy-7-trifluoromethyl-β-carboline (6 mg) in xylenes (1 ml) was treated with Pd/C (10%, 7 mg) and the mixture heated at 50 °C for one week. The reaction was concentrated after filtering it through celite, and the residue was chromatographed (1:1 hexane-ethyl acetate) on silica gel to give 1 mg of 1-hydroxy-7-trifluoromethyl-β-carboline.

A portion of dihydro 1-hydroxy-7-trifluoromethyl-β-carboline (25 mg) in THF (1 ml) was treated with a solution of lithium aluminum hydride in THF (1M, 0.5 ml). The reaction was stirred at 60 °C for 6h before quenching with water (5 ml) and extracting with ethyl acetate (3 times 10 ml). The combined organic layers were dried (MgSO₄) and concentrated to provide tetrahydro 7-trifluoromethyl-β-carboline. This material was taken up in xylenes (5 ml) and treated with Pd/C (10%, 15 mg). The mixture was stirred at 150 °C for 48h before filtering through celite and concentrating. The residue was chromatographed (ethyl acetate) on silica gel to give 5 mg of 7-tri-fluoromethyl-β-carboline.

### Example 43 7-chloro-9-(methylamino)-β-carboline trifluoroacetate

A solution of the product of example 15 (50 mg, 0.23 mmol) in AcOH/methanol (1 %, 3 ml) was treated with sodium cyanoborohydride (30 mg, 0.46 mmol) followed by formaldehyde (37%, 0.017 ml, 0.23 mmol). The reaction was stirred at RT for 36 h and then diluted with saturated NaHCO₃ (9 ml). After stirring for 15 min, the crude product was filtered and washed with water. The crude product was purified as described in example 46. The pure fractions with product were combined and lyophilized to provide 13 mg of the title compound.

Example 44 7-chloro-9-(dimethylamino)-β-carboline trifluoroacetate A solution of the product of example 15 (50 mg, 0.23 mmol) in AcOH/methanol (1 %, 3 ml) was treated with sodium cyanoborohydride (30 mg, 0.46 mmol) followed by formaldehyde (37%, 0.060 ml, 0.69 mmol). The reaction was stirred at RT for 36 h and then diluted with saturated NaHCO₃ (9 ml). After stirring for 15 min, the crude product was filtered and washed with water. The crude product was purified as described in example 46. The pure fractions with product were combined and lyophilized to provide 40 mg of the title compound.

### Example 45 7-chloro-9-(methylsulfonylamino)-β-carboline trifluoroacetate

A solution of the product of example 15 (30 mg, 0.14 mmol) in pyridine (0.5 ml) was treated with methanesulfonyl chloride (0.024 ml, 0.30 mmol) in two portions over 30h. The reaction was diluted with water (5 ml) and the crude product collected and washed with water (several times). The crude product was purified as described in example 46. The pure fractions with product were combined and lyophilized to provide 16 mg of the title compound.

### Example 46 7-chloro-9-(propyionylamino)-β-carboline trifluoroacetate

A solution of the product of example 15 (30 mg, 0.14 mmol) in pyridine (1.0 ml) was treated with propionyl chloride (0.015 ml, 0.17 mmol). After stirring at RT for 4h, the reaction was diluted with water (9 ml) and saturated NaHCO₃ (1 ml). The crude product was collected and washed with water (several times). The crude product was purified by preparative HPLC using a C₁₈-packed column and eluting with a gradient (5:95 to 50:50) of water-acetonitril (with 0.1 % trifluoroacetic acid). The pure fractions with product were combined and lyophilized to provide 21 mg of the title compound.

### Example 47 7-chtoro-9-(benzoylamino)-β-carboline trifluoroacetate

A solution of the product of example 15 (30 mg, 0.14 mmol) in pyridine (1.0 ml) was treated with benzoyl chloride (0.020 ml, 0.17 mmol). After stirring at RT for 4h, the reaction was diluted with water (9 ml) and saturated NaHCO₃ (1 ml). The crude product was collected and washed with water (several times). The crude product was purified as described in example 46. The pure fractions with product were combined and lyophilized to provide 12 mg of 7-chloro-9-(benzoylamino)-β-carboline trifluoroacetate.

### Example 48 7-chloro-9-(Acetyl-methylamino)-β-carboline trifluoroacetate

A solution of the product of example 43 (19 mg, 0.082 mmol) in pyridine (0.40 ml) was treated with acetic anhydride (0.037 ml, 0.36 mmol) in two portions over 48h. The reaction was subsequently concentrated to dryness and the residue coevaporated with AcOH under reduced pressure. The crude product was purified as described in example 46. The pure fractions with product were combined and lyophilized to provide 9 mg of the title compound.

### Example 49 7-chloro-9-(4-fluorobenzoylamino)-β-carboline trifluoroacetate

A solution of the product of example 15 (30 mg, 0.14 mmol) in pyridine (1.0 ml) was treated with 4-fluorobenzoyl chloride (0.018 ml, 0.17 mmol). After stirring at RT for 18h, the reaction was diluted with water (10 ml). The crude product was purified as described in example 46. The pure fractions with product were combined and lyophilized to provide 13 mg of the title compound.

### Example 50

A cold (3 °C to 5 °C) solution of the product of example 15 (30 mg, 0.14 mmol) and pyridine (0.014 ml, 0.17 mmol) in THF (0.7 ml) was treated with phenyl chloroformate (0.018 ml, 0.145 mmol). After stirring at RT for 2h, the reaction was partitioned in ethyl acetate and buffer (pH 7.2). The organic layer was dried (brine, Na2SO₄) and concentrated to give 43 mg of phenyl carbamate. To a solution of phenyl carbamate (30 mg, 0.089 mmol) in DMSO (0.5 ml) was added 2-methoxyethylamine (0.010 ml, 0.10 mmol). After stirring at RT for 30 min, the crude reaction mixture was purified as described in example 46. The pure fractions with product were combined and lyophilized to provide 21 mg of the title compound.

### Example 51 7-chloro-9-(methoxyacetylamino)-β-carboline trifluoroacetate

A solution of the product of example 15 (35 mg, 0.16 mmol) in pyridine (1.0 ml) was treated with methoxyacetyl chloride (0.016 ml, 0.18 mmol). After stirring at RT for 2h, the reaction was diluted with water (10 ml). The crude product was purified as described in example 46. The pure fractions with product were combined and lyophilized to provide 32 mg of the title compound.

### Example 52 7-chloro-9-(3-methoxybenzoxylamino)-β-carboline trifluoroacetate

A solution of the product of example 15 (30 mg, 0.14 mmol) in pyridine (1.0 ml) was treated with m-anisoyl chloride (0.027 ml, 0.19 mmol) in two portions over 6h. The reaction was subsequently diluted with water (10 ml) and the crude product was purified as described in example 46. The pure fractions with product were combined and lyophilized to provide 33 mg of the title compound.

### Example 53 7-chloro-9-(4-methoxybenzoxylamino)-β-carboline trifluoroacetate

A solution of the product of example 15 (30 mg, 0.14 mmol) in pyridine (1.0 ml) was treated with p-anisoyl chloride (37 mg, 0.22 mmol) in two portions over 24h. The reaction was subsequently diluted with water (10 ml) and the crude product was purified as described in example 46. The pure fractions with product were combined and lyophilized to provide 24 mg of the title compound.

### Example 54 7-chloro-9-(methylcarbamylamino)-β-carboline trifluoroacetate

A solution of the product of example 15 (30 mg, 0.14 mmol) in pyridine (1.0 ml) was treated with p-anisoyl chloride (0.017 ml, 0.21 mmol) in two portions over 4h. The reaction was subsequently diluted with water (10 ml) and the crude product was purified as described in example 46. The pure fractions with product were combined and lyophilized to provide 35 mg of the title compound.

### Example 55 7-chloro-9-(isovalerylamino)-β-carboline trifluoroacetate

A solution of the product of example 15 (35 mg, 0.16 mmol) in pyridine (1.0 ml) was treated with isovalerylchloride (0.033 ml, 0.28 mmol) in two portions over 24h. The reaction was subsequently diluted with water (10 ml) and the crude product was purified as described in example 46. The pure fractions with product were combined and lyophilized to provide 52 mg of the title compound.

### Example 60 N-(6-Chloro-9H-β-carbolin-8-yl)-nicotinamide

To a solution of norharmane (2.0 g, 11.9 mmol) in water (89 ml) and 1 M aqueous HCl (29.8 ml, 29.8 mmol) was added N-chlorosuccinimide (3.17 g, 23.8 mmol) portionwise. The resulting solution was stirred at RT for 6 h, and then at 0 °C to 5 °C for 12 h. The reaction was diluted with water (100 ml) and basified cautiously with solid K₂CO₃ (4.3 g). After stirring at RT for 1 h, the product was collected and washed with water. The crude product was refluxed in chloroform for 1 h and filtered after cooling to 15 °C to provide 2.05 g of 7-chloro-β-carboline.

A mixture 7-chloro-β-carboline (500 mg, 2.48 mmol) in concentrated nitric acid (20 ml) was stirred at RT for 22 h. The reaction mixture was carefully poured into cold (3 °C to 5 °C) water (50 ml), and after stirring for 2 h the precipitate was collected. The solid was suspended in saturated aqueous NaHCO₃ (50 ml) and stirred at RT for 12 h. The product was filtered and washed with water to provide 550 mg of 7-chloro-9-nitro-β-carboline.

To a suspension of 7-chloro-9-nitro-β-carboline (548 mg, 2.22 mmol) in EtOH (14 ml) at 65 °C to 70 °C was added tin chloride dihydrate (2.5 g, 11.1 mmol). Thereafter, 6M aqueous HCl (14 ml) was added dropwise. The mixture was stirred at 70 °C to 80 °C for 3.5 h and then partitioned slowly into saturated aqueous NaHCO₃ (150 ml) and EtOAc (100 ml). The aqueous phase was extracted (2 times) and the combined organic solutions dried (brine; NaSO₄) and concentrated to give 484 mg of 9-amino-7-chloro-β-carboline.

To a cold (3-5 °C) solution of 9-amino-7-chloro-β-carboline (2.75 g, 12.7 mmol) in pyridine (150 ml) was added nicotinyl chloride hydrochloride (2.82 g, 15.8 mmol). The reaction was allowed to warm to RT and stirred for 20 h before diluting the reaction with water (100 ml) and 1 M NaOH (25 ml). After stirring for 1 h at RT, the mixture was poured into water (200 ml). The mixture was allowed to stand for 1 h and the product was filtered to provide 3.80 g of the title compound after washing with water and drying under reduced pressure at RT.

### Example 68 N-(6-Chloro-7-methoxy-9H-β-carbolin-8-yl)-nicotinamide

A mixture of 6-methoxytryptamine (9.10 g, 47.8 mmol) in EtOAc (40 ml) and pH 4.5 NaOAc buffer (40 ml) was treated with glyoxalic acid hydrate (5.30, 57.6 mmol). The mixture was stirred vigorously for 2 days and then diluted with hexane (40 ml). The product was filtered and washed with water and (1:1) hexane-ethyl acetate. The crude product was crystallized from methanol after filtration of a hot methanol solution.

The crude product (11.5 g) from above in 6N HCl (100 ml) was treated 3 times with concentrated HCl (5.0 ml) every 15 min while refluxing. After refluxing for a total of 1 h, the reaction was concentrated to give a residue. This residue was slurried and sionicated with 10% Na₂CO₃ (300 ml) and filtered to give the free amine (7.20 g). The above amine was refluxed in xylenes (200 ml) and pyridine (100 ml) with 10% Pd/C (3 g) for 5 h. The hot reaction was filtered thru celite and the filtrate was concentrated to give 6.38 g of 8-methoxy-carboline.

To a mixture of 8-methoxy-β -carboline (1.0 g, 5 mmol) in THF (100 ml) was added N-chlorosuccinimide (0.70 g, 5.2 mmol). The reaction was stirred at RT for 4 h before concentrating and washing the residue with 1:1:1 mixture of 10% Na₂CO₃, hexane, and EtOAc (400 ml). The resulting residue was triturated with xylenes to provide 677 mg of 7-chloro-8-methoxy-β-carboline.

A solution of 7-chloro-8-methoxy-β-carboline (677 mg, 2.9 mmol) in trifluoroacetic acid (10 ml) was treated with NaNO₃ (260 mg, 3.06 mmol). The reaction was stirred for 3 h at RT and then concentrated. The crude product was chromatographed on silica eluting with a 5 % to 10% methanol in chloroform gradient to provide 463 mg of 7-chloro-8-methoxy-9-nitro-β-carboline.

A solution of 7-chloro-8-methoxy-9-nitro-β-carboline (460 mg, 1.66 mmol) in EtOH (25 ml) was treated with SnCl₂-2 H₂O (450 mg, 2.00 mmol). The reaction was stirred for 5 h at 65 °C and then concentrated. The crude product was chromatographed on silica eluting with a 5 to 10% methanol in chloroform gradient to provide 410 mg of 7-chloro-8-methoxy-9-nitro-β-carboline.

A solution of 7-chloro-8-methoxy-9-nitro-β-carboline (21 mg, 0.085 mmol) in pyridine (1 ml) was treated with nicotinyl chloride hydrochloride (54 mg, 0.30 mmol) and 4-dimethylaminopyridine (5 mg). After stirring at 95 °C - 100 °C for 7 h the reaction was concentrated, the residue slurried with 10% Na₂CO₃, and then chromatographed on silica eluting with a 5 % to 10% methanol in chloroform gradient to provide 4.7 mg of the title compound.

### Example 82 N-(6-Chloro-9H-β-carbolin-8-yl)-3,4-difluoro-benzamide

To a cold (3°C - 5°C) solution of 9-amino-7-chloro-β-carboline (2.50 g, 11.5 mmol, as prepared in example 60 above) in pyridine (130 ml) was added 3,4-diflourobenzoyl chloride (1.67 ml, 13.25 mmol). The reaction was allowed to warm to RT and stirred for 20 h before diluting the reaction with water (60 ml) and 1 M NaOH (15 ml). After stirring for 3 h at RT, the pH of the mixture was adjusted to 8-9 with 1 M HCl and then poured into water (250 ml). The mixture was allowed to stand for 30 min and the product was filtered to provide 3.95 g of the title compound after washing with water and drying under reduced pressure at 55 °C - 60 °C.

### Example 83 6-Chloro-N-(6-chloro-9H-β-carbolin-8-yl)-nicotinamide

To a cold (3-5 °C) solution of 9-amino-7-chloro-β-carboline (1.40 g, 6.45 mmol, as prepared in example 60 above) in pyridine (72 ml) was added 6-chloro-nicotinyl chloride (1.30 g, 7.42 mmol). The reaction was allowed to warm to RT and stirred for 16 h before diluting the reaction with water (60 ml) and 1 M NaOH (8 ml). After stirring for 40 min at RT, the mixture was poured into water (200 ml). The mixture was allowed to stand for 30 min and the product was filtered to provide 2.20 g of the title compound after washing with water and drying under reduced pressure at RT.

The examples in Table 1 show the structures of the prepared compounds and were prepared according to the previous examples.

**Table 1:**

| Example | Structure | Empirical formula | MS (M+ H) |
|---|---|---|---|
| 1 | 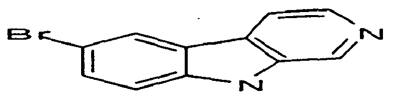 | C₁₁H₇BrN₂ | 248 |
| 2 | 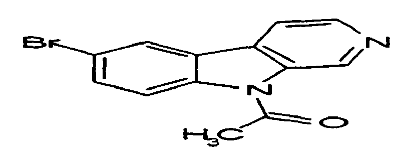 | C₁₃H₉BrN₂O | 290 |
| 3 | 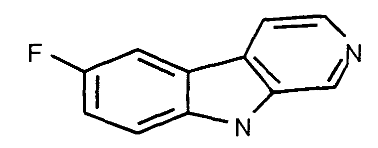 | C₁₁H₇FN₂ | 187 |
| 4 | 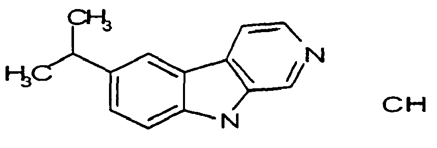 | C₁₄H₁₅ClN₂ | 211 |
| 5 | 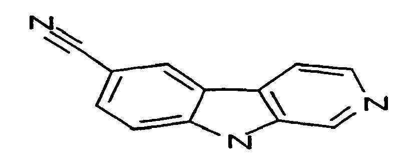 | C₁₂H₇N₃ | 194 |
| 6 | 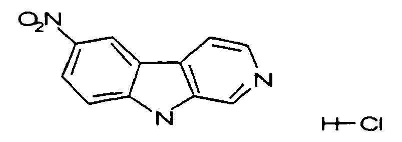 | C₁₁H₈ClN₃O₂ | 214 |
| 7 | 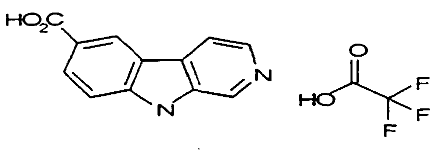 | C₁₄H₉F₃N₂O₄ | 213 |
| 8 | 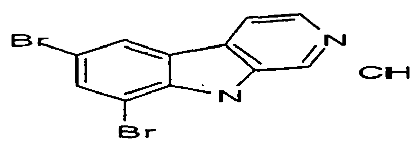 | C₁₁H₇Br₂ClN₂ | 327 |
| 9 | 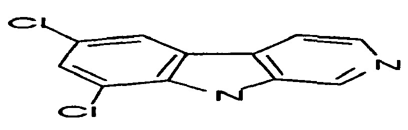 | C₁₁H₆Cl₂N₂ | 238 |
| 10 | 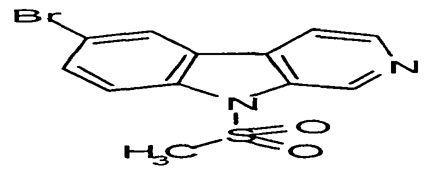 | C₁₂H₉BrN₂O₂S | 326 |
| 11 | 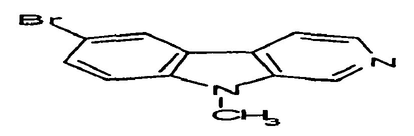 | C₁₂H₉BrN₂ | 262 |
| 12 | 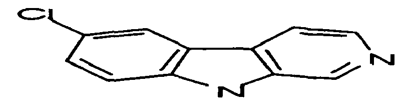 | C₁₁H₇ClN₂ | 204 |
| 13 | 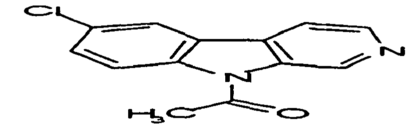 | C₁₃H₉ClN₂O | 246 |
| 14 | 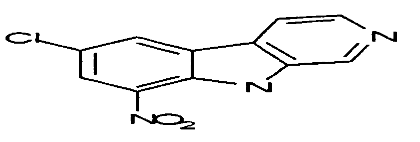 | C₁₁H₆ClN₃O₂ | 249 |
| 15 | 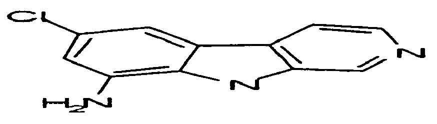 | C₁₁H₈ClN₃ | 219 |
| 16 | 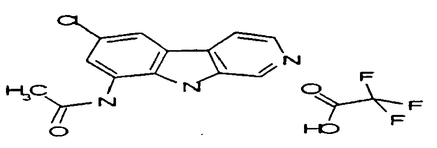 | C₁₅H₁₁ClF₃N₃O₃ | 261 |
| 17 | 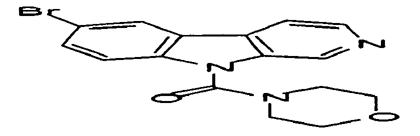 | C₁₆H₁₄BrN₃O₂ | 361 |
| 18 | 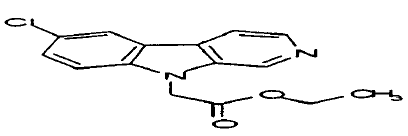 | C₁₅H₁₃ClN₂O₂ | 290 |
| 19 | 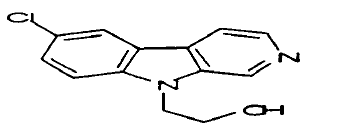 | C₁₃H₁₁ClN₂O | 248 |
| 20 | 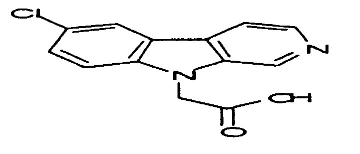 | C₁₃H₉ClN₂O₂ | 262 |
| 21 | 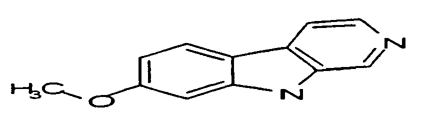 | C₁₂H₁₀N₂O | 199 |
| 22 | 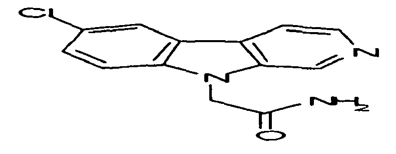 | C₁₃H₁₀ClN₃O | 261 |
| 23 | 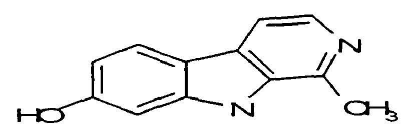 | C₁₂H₁₀N₂O | 199 |
| 24 | 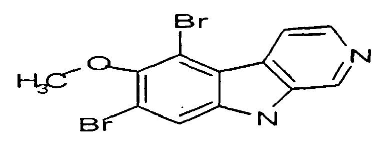 | C₁₂H₈Br₂N₂O | 257 |
| 25 | 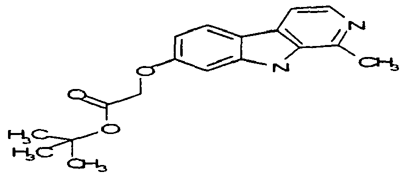 | C₁₈H₂₀N₂O₃ | 313 |
| 26 | 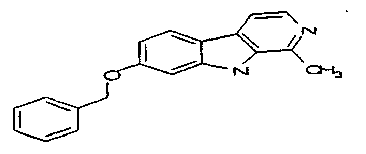 | C₁₉H₁₆N₂O | 289 |
| 27 | 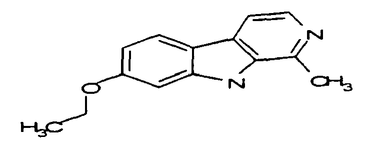 | C₁₄H₁₄N₂O | 227 |
| 28 | 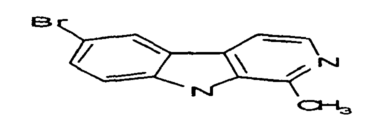 | C₁₂H₉BrN₂ | 262 |
| 29 | 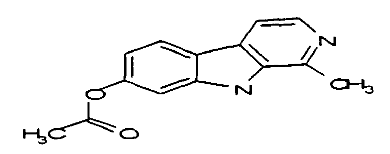 | C₁₄H₁₂N₂O₂ | 241 |
| 30 | 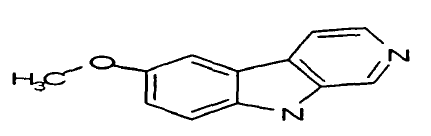 | C₁₂H₁₀N₂O | 199 |
| 31 | 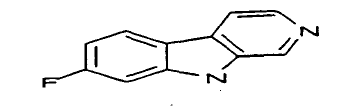 | C₁₁H₇FN₂ | 187 |
| 32 | 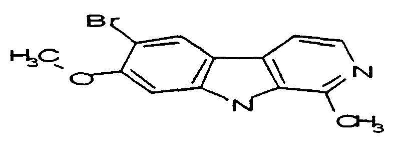 | C₁₃H₁₁BrN₂O | 292 |
| 33 | 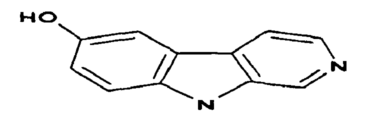 | C₁₁H₈N₂O | 185 |
| 34 | 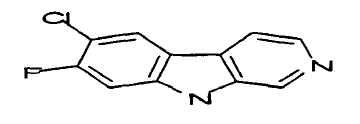 | C₁₁H₆ClFN₂ | 222 |
| 35 | 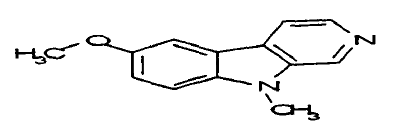 | C₁₃H₁₂N₂O | 213 |
| 36 | 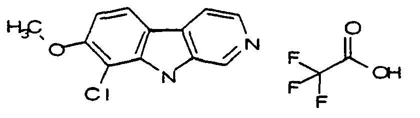 | | 348 |
| 37 | 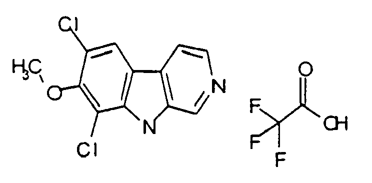 | | 382 |
| 38 | 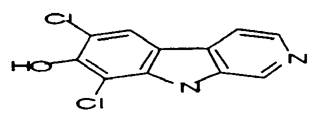 | | 254 |
| 39 | 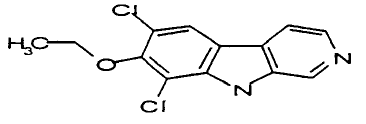 | | 282 |
| 40 | 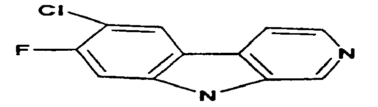 | | 222 |
| 41 | 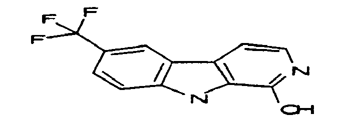 | | 253 |
| 42 | 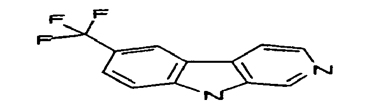 | | 237 |
| 43 | 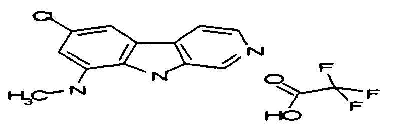 | | 347 |
| 44 | 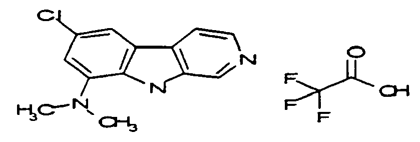 | | 361 |
| 45 | 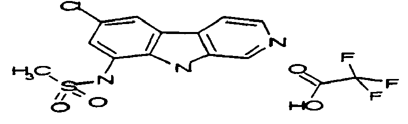 | | 411 |
| 46 | 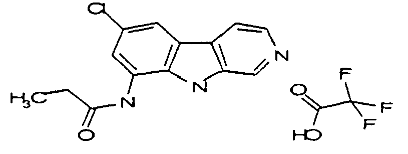 | | 389 |
| 47 | 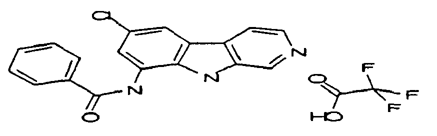 | | 437 |
| 48 | 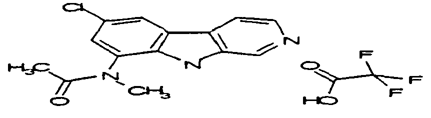 | | 388 |
| 49 | 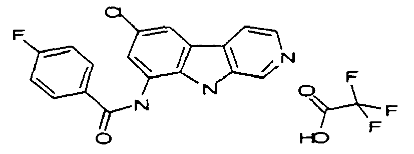 | | 454 |
| 50 | 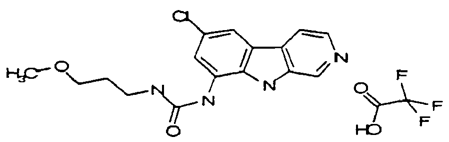 | | 448 |
| 51 | 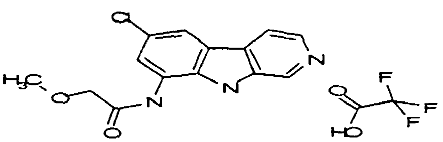 | | 404 |
| 52 | 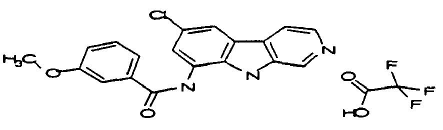 | | 467 |
| 53 | 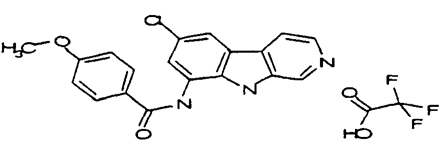 | | 467 |
| 54 | 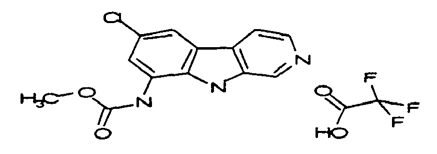 | | 391 |
| 55 | 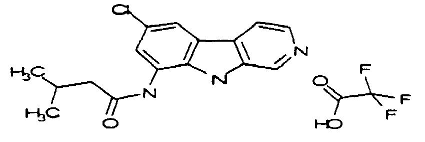 | | 416 |
| 56 | 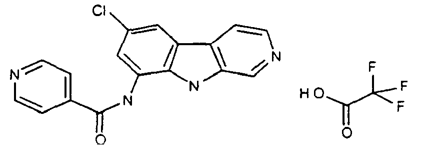 | C₁₉H₁₂ClF₃N₄O₃ | 323 |
| 57 | 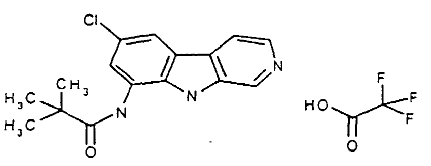 | C₁₈H₁₇ClF₃N₃O₃ | 302 |
| 58 | 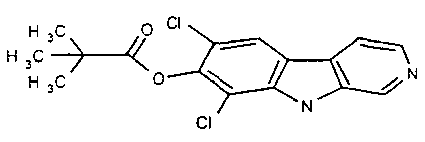 | C₁₆H₁₄Cl₂N₂O₂ | 338 |
| 59 | 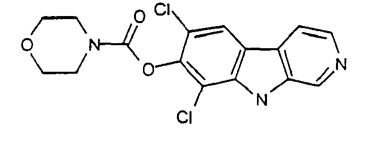 | C₁₆H₁₃Cl₂N₃O₃ | 367 |
| 60 | 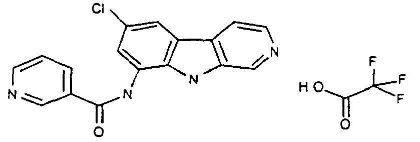 | C₁₉H₁₂ClF₃N₄O₃ | 323 |
| 61 | 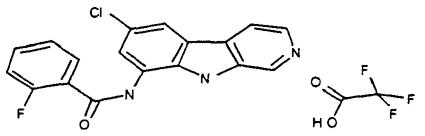 | C₂₀H₁₂ClF₄N₃O₃ | 340 |
| 62 | 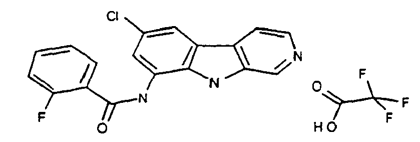 | C₂₀H₁₂ClF₄N₃O₃ | 340 |
| 63 | 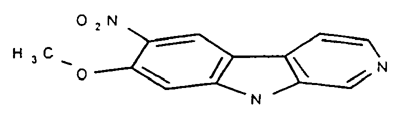 | C₁₂H₉N₃O₃ | 244 |
| 64 | 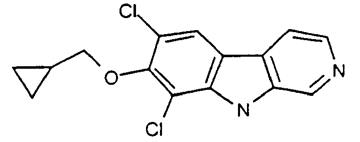 | C₁₅H₁₂Cl₂N_{2O} | 308 |
| 65 | 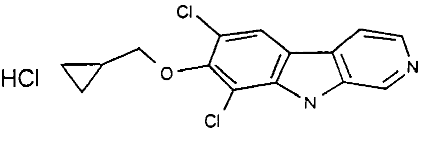 | C₁₅H₁₃Cl₃N₂O | |
| 66 | 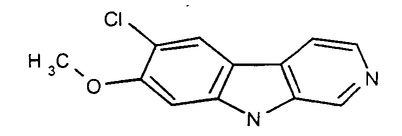 | C₁₂H₉ClN₂O | 234 |
| 67 | 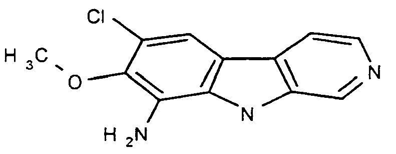 | C₁₂H₁₀ClN₃O | 249 |
| 68 | 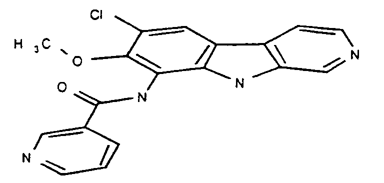 | C₁₈H₁₃ClN₄O₂ | 353 |
| 69 | 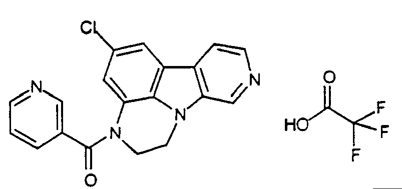 | C₂₁H₁₄ClF₃N₄O₃ | 349 |
| 70 | 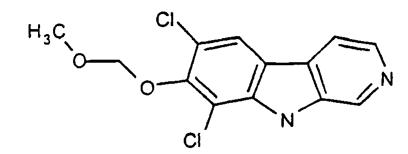 | C₁₃H₁₀Cl₂N₂O₂ | 298 |
| 71 | 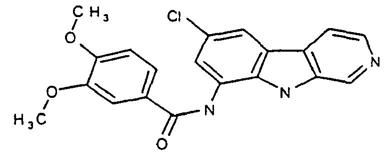 | C₂₀H₁₆ClN₃O₃ | 383 |
| 72 | 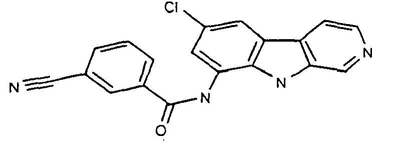 | C₁₉H₁₁ClN₄O | 348 |
| 73 | 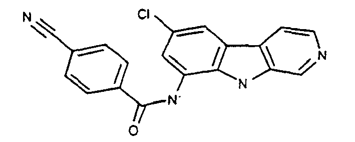 | C₁₉H₁₁ClN₄O | 348 |
| 74 | 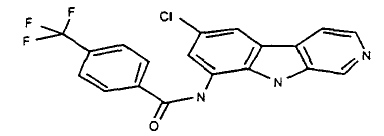 | C₁₉H₁₁ClF₃N₃O | 391 |
| 75 | 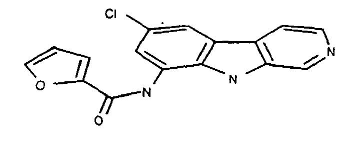 | C₁₆H₁₀ClN₃O₂ | 313 |
| 76 | 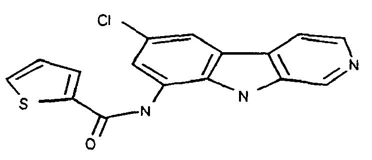 | C₁₆H₁₀ClN₃OS | 329 |
| 77 | 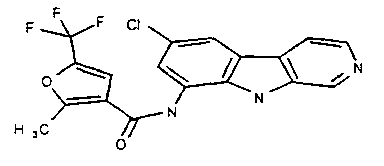 | C₁₈H₁₁ClF₃N₃O₂ | 395 |
| 78 | 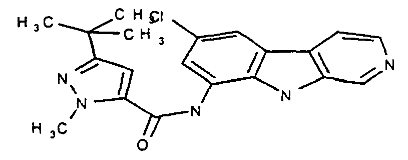 | C₂₀H₂₀ClN₅O | 383 |
| 79 | 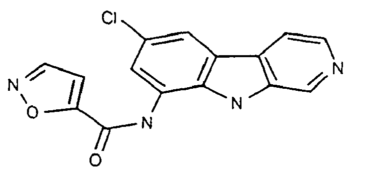 | C₁₅H₉ClN₄O₂ | 314 |
| 80 | 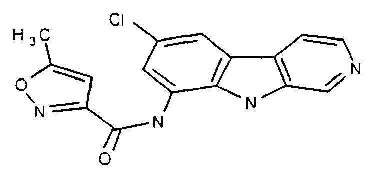 | C₁₆H₁₁ClN₄O₂ | 328 |
| 81 | 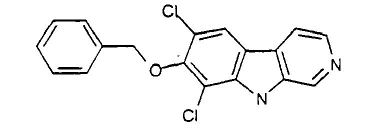 | C₁₈H₁₂Cl₂N₂O | 344 |
| 82 | 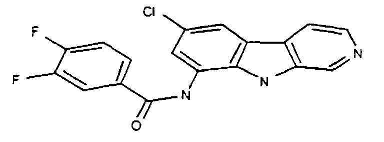 | C₁₈H₁₀ClF₂N₃O | 359 |
| 83 | 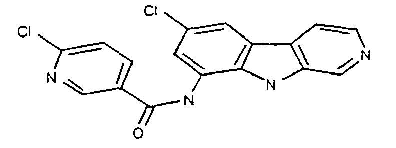 | C₁₇H₁₀Cl₂N₄O | 358 |
| 84 | 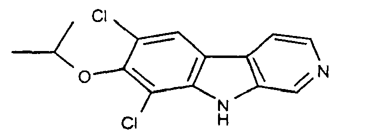 | C₁₄H₁₂Cl₂N₂O | 296 |
| 85 | 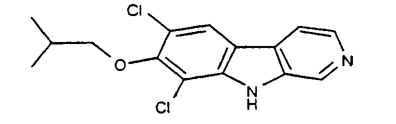 | C₁₅H₁₄Cl₂N₂O | 310 |
| 86 | 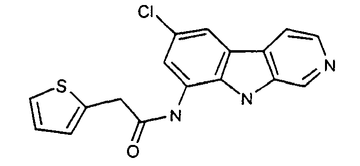 | C₁₇H₁₂ClN₃OS | 343 |
| 87 | 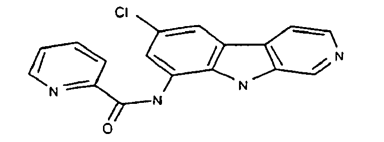 | C₁₇H₁₁ClN_{4O} | 323 |
| 88 | 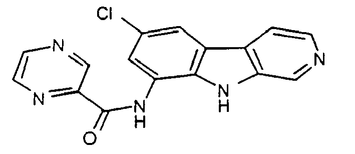 | C₁₆H₁₀ClN₅O | 323 |
| 89 | 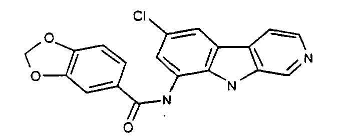 | C₁₉H₁₂ClN₃O₃ | 367 |
| 90 | 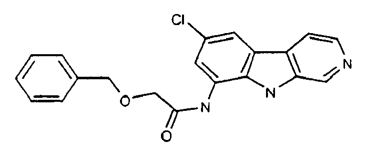 | C₂₀H₁₆ClN₃O₂ | 366 |
| 91 | 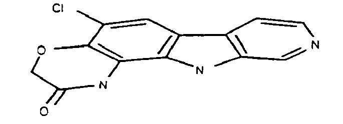 | C₁₃H₈ClN₃O₂ | 274 |
| 92 | 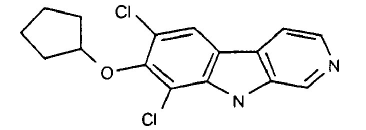 | C₁₆H₁₄Cl₂N₂O | 322 |
| 93 | 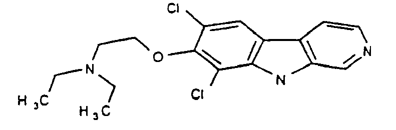 | C₁₇H₁₉Cl₂N₃O | 353 |
| 94 | 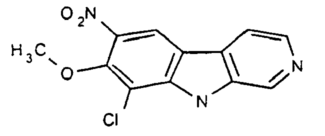 | C₁₂H₈ClN₃O₃ | 279 |
| 95 | 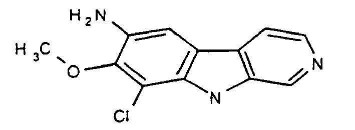 | C₁₂H₁₀ClN₃O | 248 |
| 96 | 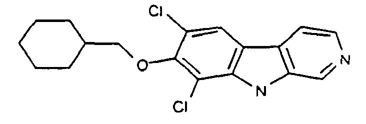 | C₁₈H₁₈Cl₂N₂O | 350 |
| 97 | 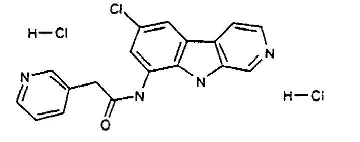 | C₁₈H₁₅Cl₃N₄O | 337 |
| 98 | 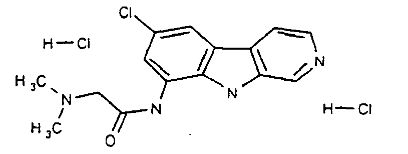 | C₁₅H₁₇Cl₃N₄O | 290 |
| 99 | 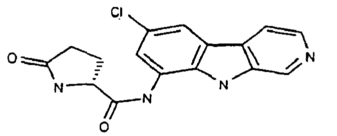 | C₁₆H₁₃ClN₄O₂ | 330 |
| 100 | 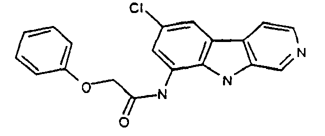 | C₁₉H₁₄ClN₃O₂ | 353 |
| 101 | 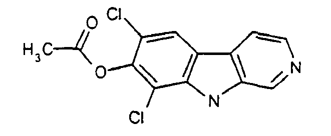 | C₁₃H₈Cl₂N₂O₂ | 296 |
| 102 | 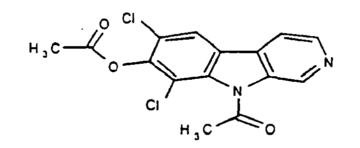 | C₁₅H₁₀Cl₂N₂O₃ | 338 |
| 103 | 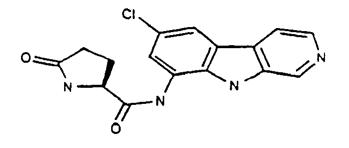 | C₁₆H₁₃ClN₄O₂ | 330 |
| 104 | 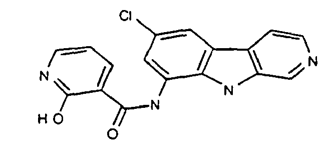 | C₁₇H₁₁ClN₄O₂ | 340 |
| 105 | 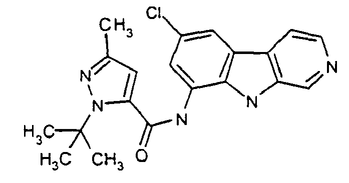 | C₂₀H₂₀ClN₅O | 383 |
| 106 | 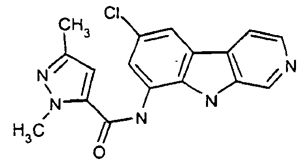 | C₁₇H₁₄ClN₅O | 341 |
| 107 | 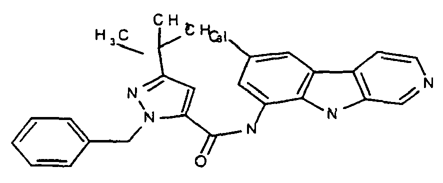 | C₂₆H₂₄ClN₅O | 359 |
| 108 | 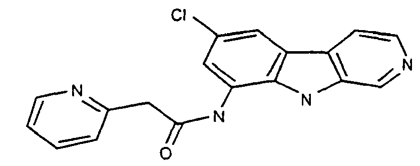 | C₁₈H₁₃ClN₄O | 338 |
| 109 | 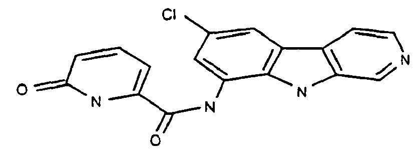 | C₁₇H₁₁ClN₄O₂ | 340 |
| 110 | 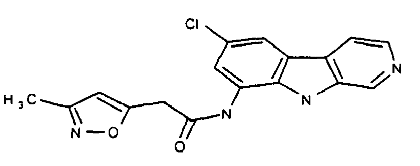 | C₁₇H₁₃ClN₄O₂ | 342 |
| 111 | 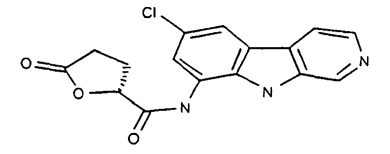 | C₁₆H₁₂ClN₃O₃ | 331 |
| 112 | 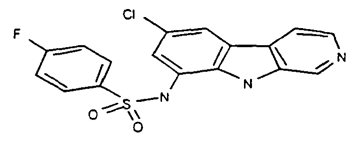 | C₁₇H₁₁ClFN₃O₂S | 377 |
| 113 | 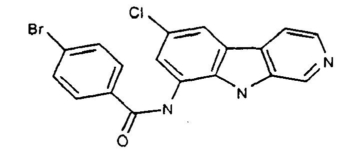 | C₁₈H₁₁BrClN₃O | 401 |
| 114 | 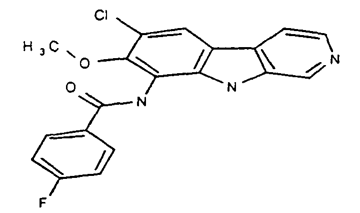 | C₁₉H₁₃ClFN₃O₂ | 371 |
| 115 | 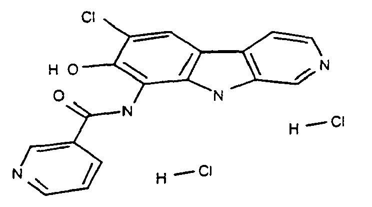 | C₁₇H₁₃Cl₃N₄O₂ | 339 |
| 116 | 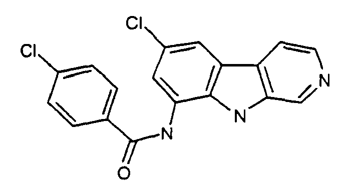 | C₁₈H₁₁Cl₂N₃O | 357 |
| 117 | 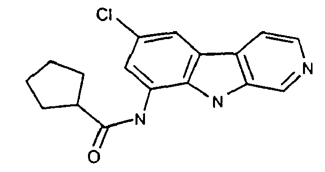 | C₁₇H₁₆ClN₃O | 314 |
| 118 | 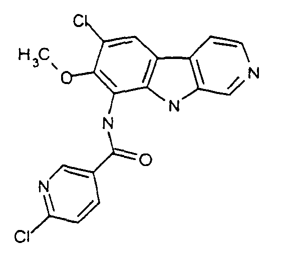 | C₁₈H₁₂Cl₂N₄O₂ | 388 |
| 119 | 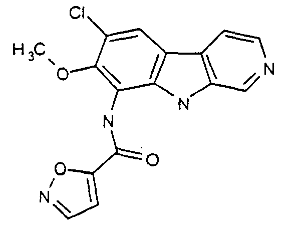 | C₁₆H₁₁ClIN₄O₃ | 343 |
| 120 | 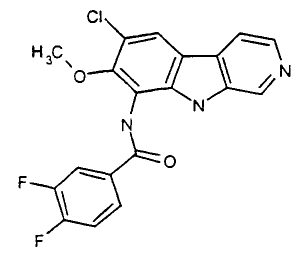 | C₁₉H₁₂ClF₂N₃O₂ | 390 |
| 121 | 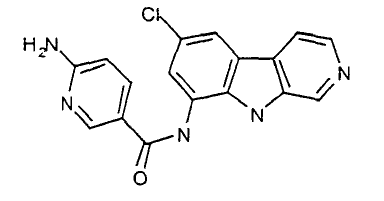 | C₁₇H₁₂ClN₅O | 339 |

### Pharmacological examples

### IₖB-kinase ELISA

The in-vitro assay for detecting and measuring inhibition activity against IkBα-kinase complex by candidate pharmacological agents employs a biotinylated polypeptide spanning both Ser³² and Ser³⁶ of IkBα and a specific antibody binding only to the phosphorylated form of the polypeptide, being either monoclonal or polyclonal (such as the commercially-available anti-phospho-serine³² IkBα antibodies from New England Biolabs, Beverly, MA, USA, cat. #9240). Once the antibody-phospho-polypeptide complex is formed, the complex can be detected by a variety of analytical methods utilizing such as radioactivity, luminescence, fluorescence or optical absorbance. For the use of the ELISA method, the complex can be immobilized either onto a biotin-binding plate (such as Neutravidin coated plate) and detected with a secondary antibody conjugated to HRP, or onto an antibody-binding plate (such as Protein-A coated plate) and detected with biotin-binding protein conjugated to HRP (such as Streptavidin-HRP). The level of activity can be correlated with a standard curve using synthetic phosphopeptides corresponding to the substrate polypeptide.

### Experimental

IkBα kinase complex was prepared by first diluting 10 mL of HeLa S3 cell-extracts S100 fraction with 40 mL of 50 mM HEPES pH 7.5. Then, 40% ammonium sulfate was added and incubated on ice for 30 minutes. Precipitated pellet was redissolved with 5 mL of SEC buffer (50 mM HEPES pH 7.5, 1 mM DTT, 0.5 mM EDTA, 10 mM 2-glycerophosphate), clarified by centrifugation at 20,000 x g for 15 min., and filtration through a 0.22 µm filter unit. Sample was loaded onto a 320-mL Superose-6 FPLC column (Amersham Pharmacia Biotech AB, Uppsala, Sweden) equilibrated with SEC buffer operated at 2 mUmin flow rate at 4°C. Fractions spanning the 670-kDa molecular-weight marker were pooled for activation. Kinase-containing pool was then activated by incubating with 100 nM MEKK1Δ, 250 µM MgATP, 10 mM MgCl₂, 5 mM DTT, 10 mM 2-glycerophosphate, 2.5 µM Microcystin-LR, for 45 minutes at 37°C. Activated enzyme was stored at -80°C until further use. Per well of a 96-well plate, compounds at various concentrations in 2 µL DMSO were pre-incubated for 30 minutes at 25°C with 43 µL of activated enzyme diluted [1:25] with assay buffer (50 mM HEPES pH 7.5, 5 mM DTT, 10 mM MgCl₂, 10 mM 2-glycerophosphate, 2.5 µM Microcystin-LR). Five microliters of peptide substrate (biotin-(CH₂)₆- DRHDSGLDSMKD-CONH₂) at 200 µM stock solution was added to each well and incubated for 1 hour before quenching with 150 µL of 50 mM HEPES pH 7.5, 0.1% BSA, 50 mM EDTA, plus [1:200] antibody. Quenched kinase reaction samples and phospho-peptide-calibration standards (biotin-(CH₂)₆- DRHDS[PO₃]GLDSMKD-CONH₂, serially diluted in assay buffer) at 100 µL per well were transferred to a Protein-A plate (Pierce Chemical Co., Rockford, IL, USA) and incubated for 2 hours. with shaking. Following 3 washes with PBS, 100 µL of 0.5 µg/mL Streptavidin conjugated with HRP (horseradish peroxidase) diluted with 50 mM HEPES/ 0.1 % BSA, was added for 30 minutes. After 5 washes with PBS, 100 µL TMB substrate (Kirkegaard & Perry Laboratories, Gaithersburg, MD, USA) was added and color development was stopped by adding 100 µL of 0.18 M H₂SO₄. Absorbance signals were recorded at 450 nm. Calibration-curve standards were fitted by linear regression using a 4-parameter dose-response equation. Based on this standard curve, levels of kinase activity were calculated in order to determine inhibition activity of candidate pharmacological agents.

Table 3 which follows shows the results.

The compound according to example 121 shows a IC₅₀ of 1.7.

**Table 3: Kinase inhibition at a substance concentration of IC₅₀ in µM**

| Example number | IkB-kinase IC₅₀ | Example number | IkB-kinase IC₅₀ | Example number | IkB-kinase IC₅₀ | Example number | IkB-kinase IC₅₀ |
|---|---|---|---|---|---|---|---|
| 1 | 0.4 | 31 | 3.3 | 61 | 0.94 | 91 | 2.3 |
| 2 | 0.4 | 32 | 4.6 | 62 | 0.38 | 92 | 0.4 |
| 3 | 1 | 33 | 1.5 | 63 | 1.3 | 93 | 1.3 |
| 4 | 2.5 | 34 | 1 | 64 | 0.11 | 94 | 0.64 |
| 5 | 1 | 35 | 3.5 | 65 | 0.10 | 95 | 1.0 |
| 6 | 3 | 36 | 1.4 | 66 | 0.7 | 96 | 1.3 |
| 7 | 65 | 37 | 0.15 | 67 | 0.5 | 97 | 0.69 |
| 8 | 0.2 | 38 | 11 | 68 | 0.16 | 98 | 0.9 |
| 9 | 0.2 | 39 | 0.1 | 69 | 3.0 | 99 | 0.09 |
| 10 | 0.4 | 40 | 2 | 70 | 0.36 | 100 | 1.5 |
| 11 | 0.7 | 41 | 2.2 | 71 | 3.0 | 101 | 1.8 |
| 12 | 0.4 | 42 | 0.8 | 72 | 0.58 | 102 | 0.8 |
| 13 | 0.5 | 43 | 1 | 73 | 0.4 | 103 | 0.31 |
| 14 | 4 | 44 | 2 | 74 | 3.8 | 104 | 1.0 |
| 15 | 0.8 | 45 | 8.3 | 75 | 0.29 | 105 | 0.4 |
| 16 | 0.3 | 46 | 0.3 | 76 | 0.9 | 106 | 0.6 |
| 17 | 5 | 47 | 0.6 | 77 | 4.4 | 107 | 0.4 |
| 18 | 23 | 48 | 4 | 78 | 2.3 | 108 | 2.1 |
| 19 | 3 | 49 | 0.22 | 79 | 0.18 | 109 | 2.1 |
| 20 | 14 | 50 | 10 | 80 | 0.31 | 110 | 0.3 |
| 21 | 1.8 | 51 | 0.6 | 81 | 0.25 | 111 | 0.54 |
| 22 | 15 | 52 | 0.6 | 82 | 0.15 | 112 | 0.93 |
| 23 | 22 | 53 | 1.4 | 83 | 0.06 | 113 | 0.64 |
| 24 | 4.6 | 54 | 0.7 | 84 | 0.1 | 114 | 2.1 |
| 25 | 31 | 55 | 0.8 | 85 | 0.2 | 115 | 4.6 |
| 26 | 12 | 56 | 0.27 | 86 | 0.7 | 116 | 1.8 |
| 27 | 4.5 | 57 | 4.3 | 87 | 0.75 | 117 | 0.67 |
| 28 | 11 | 58 | 0.33 | 88 | 0.28 | 118 | 0.12 |
| 29 | 40 | 59 | 3.2 | 89 | 0.57 | 119 | 0.6 |
| 30 | 5.2 | 60 | 0.052 | 90 | 1.4 | 120 | 0.4 |

### Mouse heterotopic cardiac transplant model

In the mouse heterotopic cardiac transplant model across full histocompatibility barriers (BALB/c->C57BL/6 or B6/129), graft survival is typically limited to 7.3 ± 0.4 days (mean ± SD, n0 allografts) (see for example in Hancock WW, Sayegh MH, Zheng XG, Peach R, Linsley PS, Turka LA. Costimulatory function and expression of CD40-ligand, CD80 and CD86 or in vascularized murine cardiac allograft rejection. Proc Natl Acad Sci (USA) 93, 1996, 13967-13972; and Hancock W W, Buelow R, Sayegh MH, Turka LA. Antibody-induced transplant arteriosclerosis is prevented by graft expression of antioxidant and anti-apoptotic genes. Nature Med 4, 1998, 1392-1396).

The effects of oral administration of the compounds according to examples 49 and 60 were tested using 25 mg/kg/day for 14 days, starting at transplantation in said animal model. Whereas grafts in animals receiving the carrier, methylcellulose, rejected by 7 days (6.7 ± 0.8), grafts in compound 49-treated mice survived around 15 days (15.3 ± 0.6), and grafts in those given compound 60 survived for 20 days (20 ± 1). Current immunosuppressive therapies used in transplantation have limited efficacy and/or considerable toxicity. Targeting of IkB-kinase with these agents significantly prolonged allograft survival without associated toxicity.

## Claims

1. The use of the compounds of the formula I 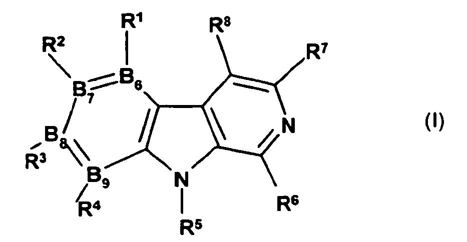 and/or a stereoisomeric form of the compounds of the formula I and/or a physiologically tolerable salt of the compounds of the formula I,
for the production of pharmaceuticals for the prophylaxis and therapy of disorders in whose course an increased activity of IₖB kinase is involved selected from asthma, osteoarthritis, rheumatoid arthritis in the case of inflammation, Alzheimer's disease, carcinomatous disorders for potentiation of cytotoxic therapies, cardiac infarct or allograft rejection,
wherein B₆, B₇, B₈ and B₉ are independently selected from the group consisting of carbon atom and nitrogen atom and wherein B₆, B₇, B₈ and B₉ together are no more than two nitrogen atoms at the same time;
where the substituents R¹, R², R³ and R⁸ independently of one another are
1. hydrogen atom,
2. halogen,
3. -OH,
4. -CN,
5. sulfo,
6. -NO₂,
7. -NH₂,
8. alkoxy,
9. substituted amino,
10. -NH-C(O)-R¹⁵, wherein R¹⁵ is a heterocycle having 5 to 7 ring atoms, alkyl, aryl, substituted aryl or substituted alkyl,
11. -COOH,
12. -O-R¹⁰, wherein R¹⁰ is alkyl, substituted alkyl or aryl,
13. -C(O)-R¹², wherein R¹² is alkyl, substituted alkyl or aryl,
14. -C(O)-O-R¹², wherein R¹² is as defined above,
15. aryl,
16. -O-aryl,
17. substituted aryl,
18. -O-substituted aryl,
19. alkyl,
20. substituted alkyl,
21. -CF₃ or
22. -CF₂-CF₃,
R4 is
1. -OH,
2. -CN,
3. sulfo,
4. -NH₂,
5. alkoxy,
6. substituted amino,
7. -NH-C(O)-R¹⁵, wherein R¹⁵ is a heterocycle having 5 to 7 ring atoms, alkyl, aryl, substituted aryl or substituted alkyl,
8. -COOH,
9. -O-R¹⁰, wherein R¹⁰ is alkyl, substituted alkyl or aryl,
10. -C(O)-O-R¹², wherein R¹² is as defined above,
11. aryl,
12. -O-aryl,
13. substituted aryl,
14. -0-substituted aryl,
15. alkyl,
16. substituted alkyl,
17. -CF₃ or
18. -CF₂-CF₃,
provided that at least one of R¹, R², R³, R⁴ and R⁸ is not a hydrogen atom,
R⁵ is
1. hydrogen atom,
2. alkyl,
3. alkyl radical, substituted at one or more positions by one or more of the radicals, halogen, amino or hydroxyl,
4. -C(O)-R⁹ or
5. -S(O)₂-R⁹, in which
R⁹ is
a) alkyl,
b) alkyl radical, substituted at one or more positions by one or more of the radicals, halogen, amino or hydroxyl,
c) aryl,
d) aryl radical, substituted at one or more positions by one or more of the radicals, halogen, amino, or hydroxyl,
e) -NH₂,
f) alkoxy or
g) substituted amino, and
R⁶ and R⁷ independently of one another are
1. hydrogen atom,
2. halogen,
3. -OH,
4. methyl,
5. -O-(C₁-C₁₀)-alkyl, wherein alkyl is unsubstituted or mono- to trisubstituted independently of one another by 5.1 aryl,
5.2 halogen,
5.3 -NO₂,
5.4 sulfo,
5.5 -COOH,
5.6 -NH₂,
5.7 -O-(C₁-C₄)-alkyl or
5.8 -OH, or
6. N(R¹³)₂, wherein R¹³ is independently of one another hydrogen atom, aryl, -C(O)-(C₁-C₄)-alkyl or substituted aryl or alkyl, wherein alkyl is unsubstituted or mono- to tri- substituted independently of one another as defined under 5.1 to 5.8, or
R¹³ together with the nitrogen atom to which it is bonded form a heterocycle having 5 to 7 ring atoms,
wherein halogen means fluorine, chlorine, bromine or iodine,
sulfo means -S(O)y-R¹⁴, wherein R¹⁴ is an alkyl, aryl, substituted aryl, substituted alkyl, amino, or substituted amino and y is zero, one or two,
alkyl means a straight or branched chain hydrocarbon radical having 1 to 10 carbon atoms,
aryl means an organic radical derived from an aromatic molecule by removal of one atom selected from phenyl, pyridyl, thiazolyl and naphthyl,
substituted aryl means an aryl radical substituted at one or more positions by one or more radicals of the group halogen, alkyl, substituted alkyl, nitro, sulfo, amino,
substituted alkyl means an alkyl radical substituted at one or more positions by one or more radicals of the group halogen, nitro, sulfo, amino, substituted amino, carboxyl, alkoxy, -O-aryl, -O-substituted aryl, and hydroxyl,
substituted amino means a -N(R¹³)₂ wherein R¹³ is independently of one another hydrogen atom, sulfo, alkyl, aryl, -C(O)-alkyl, C(O)-NH-aryl, -C(O)-O-aryl, -C(O)-O-alkyl, or C(O)-O-aryl, wherein each alkyl or aryl may be independently substituted,
alkoxy means -O-alkyl or -O-substituted alkyl, and
heterocycle having 5 to 7 ring atoms means a radical of a monocyclic saturated system having 5 to 7 ring members, which contains 1, 2 or 3 heteroatoms selected from N, O and S as ring members.

2. The use of a compound of formula II 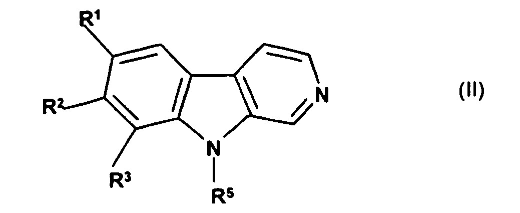 and/or a stereoisomeric form of the compounds of the formula II and/or a physiologically tolerable salt of the compounds of the formula II,
for the production of pharmaceuticals for the prophylaxis and therapy of disorders in whose course an increased activity of IₖB kinase is involved selected from asthma, osteoarthritis, rheumatoid arthritis in the case of inflammation, Alzheimer's disease, carcinomatous disorders for potentiation of cytotoxic therapies, cardiac infarct or allograft rejection, wherein;
R¹ and R² are independently from one another hydrogen atom, halogen, cyano, amino, -O-(C₁-C₄)-alkyl, nitro, -CF₃ or N(R¹³)₂,
wherein R¹³ is independently of one another hydrogen atom, -(C₁-C₇)-alkyl, -C(O)-(C₁-C₇)-alkyl, -C(O)-pyridyl, -C(O)-phenyl or -C(O)-O-(C₁-C₄)-alkyl, wherein alkyl or phenyl are unsubstituted or mono-to tri- substituted independently of one another by halogen or -O-(C₁-C₄)-alkyl,
R³ is cyano, amino, -O-(C₁-C₄)-alkyl, -CF₃ or N(R¹³)₂,
wherein R¹³ is independently of one another hydrogen atom, -(C₁-C₇)-alkyl, -C(O)-(C₁-C₇)-alkyl, -C(O)-pyridyl, -C(O)-phenyl or -C(O)-O-(C₁-C₄)-alkyl, wherein alkyl or phenyl are unsubstituted or mono-to tri- substituted independently of one another by halogen or -O-(C₁-C₄)-alkyl, and
R⁵ is hydrogen atom, -C(O)-CH₃, methyl, -S(O)₂-CH₃, -C(O)-morpholinyl, -CH₂-CH₂-OH or -CH₂-C(O)-NH₂,
provided that no more than two of R¹, R², R³ and R⁵ are a hydrogen atom.

## Patentansprüche

1. Verwendung der Verbindungen der Formel I 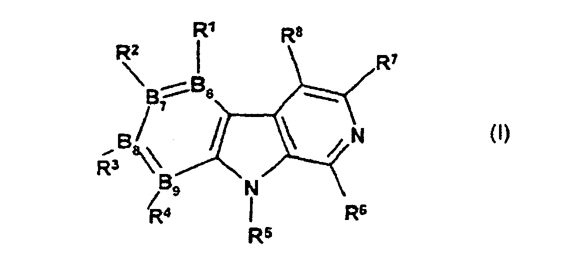 und/oder einer stereoisomeren Form der Verbindungen der Formel I und/oder eines physiologisch verträglichen Salzes der Verbindungen der Formel I zur Herstellung von Pharmazeutika zur Prophylaxe und Therapie von Störungen, an deren Verlauf eine erhöhte Aktivität von IₖB-Kinase beteiligt ist und die unter Asthma, Osteoarthritis, rheumatoider Arthritis im Fall von Entzündung, Alzheimer-Krankheit, Krebserkrankungen zur Potenzierung von cytotoxischen Therapien, Herzinfarkt oder Allotransplantatabstoßung ausgewählt sind,
wobei B₆, B₇, B₈ und B₉ unabhängig voneinander aus der Gruppe bestehend aus Kohlenstoffatom und Stickstoffatom ausgewählt sind und B₆, B₇, B₈ und B₉ zusammen für nicht mehr als zwei Stickstoffatome gleichzeitig stehen;
wobei die Substituenten R¹, R², R³ und R⁸ unabhängig voneinander für
1. Wasserstoffatom,
2. Halogen,
3. -OH,
4. -CN,
5. Sulfo,
6. -NO₂,
7. -NH₂,
8. Alkoxy,
9. substituiertes Amino,
10. -NH-C(O)-R¹⁵, worin R¹⁵ für einen Heterocyclus mit 5 bis 7 Ringatomen, Alkyl, Aryl, substituiertes Aryl oder substituiertes Alkyl steht,
11. -COOH,
12. -O-R¹⁰, worin R¹⁰ für Alkyl, substituiertes Alkyl oder Aryl steht,
13. -C(O)-R¹², worin R¹² für Alkyl, substituiertes Alkyl oder Aryl steht,
14. -C(O)-O-R¹² , worin R¹² die oben angegebene Bedeutung besitzt,
15. Aryl,
16. -O-Aryl,
17. substituiertes Aryl,
18. -O-subst.-Aryl,
19. Alkyl,
20. substituiertes Alkyl,
21. -CF₃ oder
22. -CF₂-CF₃
stehen,
R4 für
1. -OH,
2. -CN,
3. Sulfo,
4. -NH₂,
5. Alkoxy,
6. substituiertes Amino,
7. -NH-C(O)-R¹⁵, worin R¹⁵ für einen Heterocyclus mit 5 bis 7 Ringatomen, Alkyl, Aryl, substituiertes Aryl oder substituiertes Alkyl steht,
8. -COOH,
9. -O-R¹⁰, worin R¹⁰ für Alkyl, substituiertes Alkyl oder Aryl steht,
10. -C(O)-O-R¹², worin R¹² die oben angegebene Bedeutung besitzt,
11. Aryl,
12. -O-Aryl,
13. substituiertes Aryl,
14. -O-subst.-Aryl,
15. Alkyl,
16. substituiertes Alkyl,
17. -CF₃ oder
18. -CF₂-CF₃
steht,
mit der Maßgabe, daß mindestens einer der Substituenten R¹, R², R³, R⁴ und R⁸ nicht für ein Wasserstoffatom steht,
R5 für
1. Wasserstoffatom
2. Alkyl
3. Alkylrest, der in einer oder mehreren Positionen durch einen oder mehrere der Reste Halogen, Amino oder Hydroxyl substituiert ist,
4. -C(O)-R⁹ oder
5. -S(O)₂-R⁹, worin
R⁹ für
a) Alkyl,
b) Alkylrest, der in einer oder mehreren Positionen durch einen oder mehrere der Reste Halogen, Amino oder Hydroxyl substituiert ist,
c) Aryl,
d) Arylrest, der in einer oder mehreren Positionen durch einen oder mehrere der Reste Halogen, Amino oder Hydroxyl substituiert ist,
e) -NH₂,
f) Alkoxy oder
g) substituiertes Amino
steht und
R⁶ und R⁷ unabhängig voneinander für
1. Wasserstoffatom,
2. Halogen,
3. -OH,
4. Methyl,
5. -O-(C₁-C₁₀)-Alkyl, worin Alkyl unsubstituiert oder ein- bis dreifach unabhängig voneinander durch
5.1 Aryl,
5.2 Halogen,
5.3 -NO₂,
5.4 Sulfo,
5.5 -COOH,
5.6 -NH₂,
5.7 -O-(C₁-C₄)-Alkyl oder
5.8 -OH
substituiert ist, oder
6. N(R¹³)₂, worin R¹³ unabhängig voneinander für Wasserstoffatom, Aryl, -C(O)-(C₁-C₄)-Alkyl oder substituiertes Aryl oder Alkyl stehen, wobei Alkyl unsubstituiert oder ein- bis dreifach unabhängig voneinander wie unter 5.1 bis 5.8 definiert substituiert ist oder R¹³ zusammen mit dem Stickstoffatom, an das es gebunden ist, einen Heterocyclus mit 5 bis 7 Ringatomen bildet,
stehen,
wobei Halogen Fluor, Chlor, Brom oder Iod bedeutet,
Sulfo -S(O)y-R¹⁴ worin R¹⁴ für Alkyl, Aryl, substituiertes Aryl, substituiertes Alkyl, Amino oder substituiertes Amino steht und y für null, eins oder zwei steht, bedeutet,
Alkyl einen gerad- oder verzweigtkettigen Kohlenwasserstoffrest mit 1 bis 10 Kohlenstoffatomen bedeutet,
Aryl einen organischen Rest bedeutet, der sich von einem aromatischen Molekül durch Entfernung eines Atoms ableitet und unter Phenyl, Pyridyl, Thiazolyl und Naphthyl ausgewählt ist, substituiertes Aryl einen Arylrest bedeutet, der in einer oder mehreren Positionen durch einen oder mehrere Reste aus der Gruppe Halogen, Alkyl, substituiertes Alkyl, Nitro, Sulfo und Amino substituiert ist,
substitiuertes Alkyl einen Alkylrest bedeutet, der in einer oder mehreren Positionen durch einen oder mehrere Reste aus der Gruppe Halogen, Nitro, Sulfo, Amino, substituiertes Amino, Carboxyl, Alkoxy, -O-Aryl, -0-substituiertes-Aryl und Hydroxyl substituiert ist,
substituiertes Amino -N(R¹³)₂, worin R¹³ unabhängig voneinander für Wasserstoffatom, Sulfo, Alkyl, Aryl, -C(O)-Alkyl, -C(O)-NH-Aryl, -C(O)-O-Aryl, -C(O)-O-Alkyl oder -C(O)-O-Aryl, wobei jedes Alkyl oder Aryl unabhängig substituiert sein kann,
steht, bedeutet,
Alkoxy -0-Alkyl oder -O-substituiertes-Alkyl bedeutet und
Heterocyclus mit 5 bis 7 Ringatomen einen Rest eines monocyclischen gesättigten Systems mit 5 bis 7 Ringgliedern, das 1, 2 oder 3 unter N, O und S ausgewählte Heteroatome als Ringglieder enthält, bedeutet.

2. Verwendung einer Verbindung der Formel II 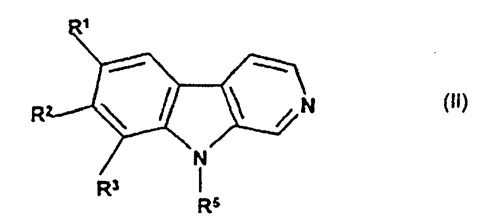 und/oder einer stereoisomeren Form der Verbindungen der Formel II und/oder eines physiologisch verträglichen Salzes der Verbindungen der Formel II
zur Herstellung von Pharmazeutika zur Prophylaxe und Therapie von Störungen, an deren Verlauf eine erhöhte Aktivität von IₖB-Kinase beteiligt ist und die unter Asthma, Osteoarthritis, rheumatoider Arthritis im Fall von Entzündung, Alzheimer-Krankheit, Krebserkrankungen zur Potenzierung von cytotoxischen Therapien, Herzinfarkt oder Allotransplantatabstoßung ausgewählt sind, wobei:
R¹ und R² unabhängig voneinander für Wasserstoffatom, Halogen, Cyano, Amino, O-(C₁-C₄)-Alkyl, Nitro, -CF₃ oder N(R¹³)₂ stehen,
wobei R¹³ unabhängig voneinander für Wasserstoffatom, -(C₁-C₇)-Alkyl, -C(O)-(C₁-C₇)-Alkyl, -C(O)-Pyridyl, -C(O)-Phenyl oder -C(O)-O-(C₁-C₄)-Alkyl, wobei Alkyl oder Phenyl unsubstituiert oder ein- bis dreifach unabhängig voneinander durch Halogen oder -O-(C₁-C₄)-Alkyl substituiert ist, steht,
R³ für Cyano, Amino, -O-(C₁-C₄)-Alkyl, -CF₃ oder N(R¹³)₂ steht, wobei R¹³ unabhängig voneinander für Wasserstoffatom, (C₁-C₇) -Alkyl, -C(O)-(C₁-C₇)-Alkyl, -C(O)-Pyridyl, -C(O)-Phenyl oder -C(O)-O-(C₁-C₄)-Alkyl, wobei Alkyl oder Phenyl unsubstituiert oder ein- bis dreifach unabhängig voneinander durch Halogen oder -O-(C₁-C₄)-Alkyl substituiert sind, steht,
R⁵ für Wasserstoffatom, -C(O)-CH₃, Methyl, -S(O)₂-CH₃ , -C(O)-Morpholinyl, -CH₂-CH₂-OH oder -CH₂-C(O)-NH₂ steht,
mit der Maßgabe, daß nicht mehr als zwei der Gruppen R¹, R², R³ und R⁵ für ein Wasserstoffatom stehen.

## Revendications

1. Utilisation des composés représentés par la formule I 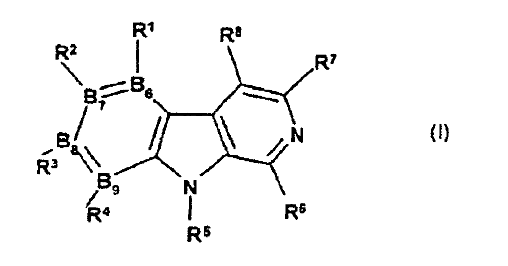 et/ou d'une forme stéréoisomère des composés représentés par la formule I et/ou d'un sel physiologiquement tolérable des composés représentés par la formule I, pour la production de produits pharmaceutiques pour la prophylaxie et la thérapie de troubles au cours desquels une activité accrue de kinase IₖB est mise en jeu choisis parmi l'asthme, l'arthrose, la polyarthrite rhumatoïde dans le cas d'une inflammation, la maladie d'Alzheimer, les troubles carcinomateux pour la potentialisation de thérapies cytotoxiques, l'infarctus du myocarde ou le rejet d'allogreffe,
où B₆, B₇, B₈ et B₉ sont indépendamment choisis dans le groupe constitué par l'atome de carbone et l'atome d'azote et où B₆, B₇, B₈ et B₉ ensemble ne sont pas plus de deux atomes d'azote en même temps ;
où les substituants R¹, R², R³ et R⁸ indépendamment les uns des autres sont
1. un atome d'hydrogène,
2. halogène,
3. -OH,
4. -CN,
5. sulfo,
6. -NO₂,
7. -NH₂,
8. alcoxy,
9. amino substitué,
10. -NH-C(O)-R¹⁵, où R¹⁵ est hétérocycle ayant 5 à 7 atomes de cycle, alkyle, aryle, aryle substitué ou alkyle substitué,
11. -COOH,
12. -O-R¹⁰, où R¹⁰ est alkyle, alkyle substitué ou aryle,
13. -C(O)-R¹² ,où R¹² est alkyle, alkyle substitué ou aryle,
14. -C(O)-O-R¹², où R¹² est tel que défini ci-dessus,
15. aryle,
16. -O-aryle,
17. aryle substitué,
18. -O-(aryle substitué),
19. alkyle,
20. alkyle substitué,
21. -CF₃ ou
22. -CF₂-CF₃,
R⁴ est
1. -OH,
2. -CN,
3. sulfo,
4. -NH₂,
5. alcoxy,
6. amino substitué,
7. -NH-C(O)-R¹⁵, où R¹⁵ est hétérocycle ayant 5 à 7 atomes de cycle, alkyle, aryle, aryle substitué ou alkyle substitué,
8. -COOH,
9. -O-R¹⁰, où R¹⁰ est alkyle, alkyle substitué ou aryle,
10. -C(O)-O-R¹², où R¹² est tel que défini ci-dessus,
11. aryle,
12. -O-aryle,
13. aryle substitué,
14. -O-(aryle substitué),
15. alkyle,
16. alkyle substitué,
17. -CF₃ ou
18. -CF₂-CF₃,
à condition qu'au moins l'un parmi R¹ R² R³ R⁴ et R⁸ ne soit pas un atome d'hydrogène,
R⁵ est
1. un atome d'hydrogène,
2. alkyle,
3. un radical alkyle, substitué en une ou plusieurs positions par un ou plusieurs des radicaux halogène, amino ou hydroxyle,
4. -C(O)-R⁹ ou
5. -S(O)₂-R⁹, où
R⁹ est
a) alkyle,
b) un radical alkyle, substitué en une ou plusieurs positions par un ou plusieurs des radicaux halogène amino ou hydroxyle,
c) aryle,
d) un radical aryle, substitué en une ou plusieurs positions par un ou plusieurs des radicaux halogène amino ou hydroxyle,
e) -NH₂,
f) alcoxy ou
g) amino substitué et
R⁶ et R⁷ indépendamment l'un de l'autre sont
1. un atome d'hydrogène,
2. halogène,
3. -OH,
4. méthyle,
5. -O-(alkyle en C₁-C₁₀), où alkyle est non substitué ou mono- à trisubstitué indépendamment à chaque fois par
5.1 aryle,
5.2 halogène,
5.3 -NO₂,
5.4 sulfo,
5.5 -COOH,
5.6 -NH₂,
5.7 -O-(alkyle en C₁-C₄) ou
5.8 -OH ou
6. N(R¹³)₂, où les R¹³ sont indépendamment l'un de l'autre un atome d'hydrogène, aryle, -C(O)-(alkyle en C₁-C₄) ou aryle substitué ou alkyle, où alkyle est non substitué ou mono-à trisubstitué indépendamment à chaque fois comme défini en 5.1 à 5.8, ou
R¹³ conjointement avec l'atome d'azote auquel il est lié forme hétérocycle ayant 5 à 7 atomes de cycle,
où halogène désigne le fluor, le chlore, le brome ou l'iode,
sulfo désigne -S(O)_{y}-R¹⁴, où R¹⁴ est alkyle, aryle, aryle substitué, alkyle substitué, amino ou amino substitué et y est zéro, un ou deux,
alkyle désigne un radical hydrocarboné à chaîne droite ou ramifiée ayant 1 à 10 atomes de carbone,
aryle désigne un radical organique dérivé d'une molécule aromatique par élimination d'un atome choisi parmi phényle, pyridyle, thiazolyle et naphtyle,
aryle substitué désigne un radical aryle substitué en une ou plusieurs positions par un ou plusieurs radicaux parmi halogène, alkyle, alkyle substitué, nitro, sulfo, amino,
alkyle substitué désigne un radical alkyle substitué en une ou plusieurs positions par un ou plusieurs radicaux parmi halogène, nitro, sulfo, amino, amino substitué, carboxyle, alcoxy, -O-aryle, -O-(aryle substitué) et hydroxyle,
amino substitué désigne un groupe -N(R¹³)₂ où les R¹³ sont indépendamment l'un de l'autre un atome d'hydrogène, sulfo, alkyle, aryle, -C(O)-alkyle, -C(O)-NH-aryle, -C(O)-O-aryle, -C(O)-O-alkyle ou -C(O)-O-aryle, où chaque alkyle ou aryle peut être indépendamment substitué,
alcoxy désigne -O-alkyle ou -0-(alkyle substitué) et hétérocycle ayant 5 à 7 atomes de cycle désigne un radical d'un système monocyclique saturé ayant 5 à 7 chaînons de cycle, qui contient 1, 2 ou 3 hétéroatomes choisis parmi N, 0 et S comme chaînons de cycle.

2. Utilisation d'un composé de formule II 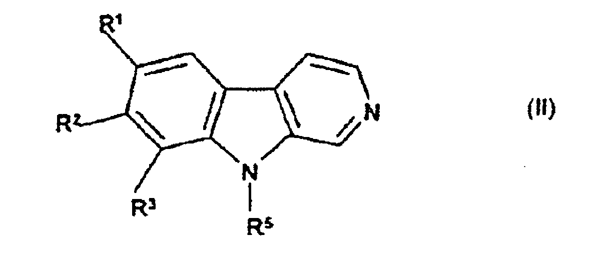 et/ou d'une forme stéréoisomère des composés représentés par la formule II et/ou d'un sel physiologiquement tolérable des composés représentés par la formule II, pour la production de produits pharmaceutiques pour la prophylaxie et la thérapie de troubles au cours desquels une activité accrue de kinase IₖB est mise en jeu choisis parmi l'asthme, l'arthrose, la polyarthrite rhumatoïde dans le cas d'une inflammation, la maladie d'Alzheimer, les troubles carcinomateux pour la potentialisation de thérapies cytotoxiques, l'infarctus du myocarde ou le rejet d'allogreffe, où
R¹ et R² sont indépendamment l'un de l'autre un atome d'hydrogène, halogène, cyano, amino, -O-(alkyle en C₁-C₄) , nitro, -CF₃ ou N(R¹³)₂,
où les R¹³ sont indépendamment l'un de l'autre un atome d'hydrogène, -(alkyle en C₁-C₇), -C(O)-(alkyle en C₁-C₇) , -C(0)-pyridyle, -C(O)-phényle ou -C(O)-O-(alkyle en C₁-C₄), où alkyle ou phényle sont non substitués ou mono- à trisubstitués indépendamment à chaque fois par halogène ou -O-(alkyle en C₁-C₄),
R³ est cyano, amino, -0-(alkyle en C₁-C₄), -CF₃ ou N(R¹³)₂,
où les R¹³ sont indépendamment l'un de l'autre un atome d'hydrogène, -(alkyle en C₁-C₇), -C(O)-(alkyle en C₁-C₇), -C(O)-pyridyle, -C(O)-phényle ou -C(O)-O-(alkyle en C₁-C₄), où alkyle ou phényle sont non substitués ou mono- à trisubstitués indépendamment à chaque fois par halogène ou -O-(alkyle en C₁-C₄) et
R⁵ est un atome d'hydrogène, -C(O)-CH₃, méthyle, -S(O)₂-CH₃ , -C(O)-morpholinyle, -CH₂-CH₂-OH ou -CH₂-C(O)-NH₂,
à condition que pas plus de deux parmi R¹, R² R³ et R⁵ soient un atome d'hydrogène.
